# EUROPEAN PATENT SPECIFICATION

(11) **EP 1 228 219 B1**
(45) Date of publication and mention of the grant of the patent: **09.11.2005**
(21) Application number: 00986056.0
(22) Date of filing: 09.11.2000
(51) Int. Cl.: C12N 15/32, C07K 14/325, C12N 15/62, A01H 5/00, A01N 63/00

(54) **BACILLUS THURINGIENSIS HYBRID TOXINS**
HYBRID-TOXINE VON BACILLUS THURINGIENSIS
TOXINES HYBRIDES DE BACILLUS THURINGIENSIS

(30) Priority: 09.11.1999 EP 99203723
(43) Date of publication of application: 07.08.2002
(73) Proprietor: Plant Research International B.V., 6708 PB Wageningen (NL)
(72) Inventor: DE MAAGD, Rudolf, Aart, NL-6708 HL Wageningen (NL); BOSCH, Hendrik, Jan, NL-3572 ZM Utrecht (NL)
(74) Representative: De Hoop, Eric
(86) International application number: PCT/NL2000/000817
(87) International publication number: WO 2001/034811

(56) References cited:
- WO-A-95/06730
- WO-A-99/50293
- VISSER ET AL: "Domain-function studies of Bacillus thuringiensis crystal proteins: a genetic approach" ENTWISTLE, P.F. (EDS) : "BACILLUS THURINGIENSIS, AN ENVIRONMENTAL BIOPESTICIDE: THEORY AND PRACTICE", 1993, pages 71-88, XP002086356 GB,CHICHESTER, WILEY & SONS
- ELY S: "THE ENGINEERING OF PLANTS TO EXPRESS BACILLUS THURINGIENSIS DELTA-ENDOTOXINS" ENTWISTLE, P.F. (EDS) : "BACILLUS THURINGIENSIS, AN ENVIRONMENTAL BIOPESTICIDE: THEORY AND PRACTICE", 1993, pages 105-124, XP002054693 GB,CHICHESTER, WILEY & SONS
- BRADLEY D ET AL: "The Insecticidal CryIB Crystal Protein of Bacillus thuringiensis ssp. thuringiensis Has Dual Specificity to Coleopteran and Lepidopteran Larvae." JOURNAL OF INVERTEBRATE PATHOLOGY, vol. 65, no. 2, 1995, pages 162-173, XP000891272 ISSN: 0022-2011 cited in the application
- MAAGD DE R A: "DIFFERENT DOMAINS OF BACILLUS THURINGIENSIS DELTA-ENDOTOXINS CAN BIND TO INSECT MIDGUT MEMBRANE PROTEINS ON LIGAND BLOTS" APPLIED AND ENVIRONMENTAL MICROBIOLOGY, vol. 62, no. 8, August 1996 (1996-08), pages 2753-2757, XP002061801 ISSN: 0099-2240 cited in the application

### SEQUENCE LISTING

<110> CPRO-DLO
<120> Bacillus thuringiensis hybrid toxins
<130> E158870
<140>
   <141>
<160> 10
<170> PatentIn Ver. 2.1
<210> 1
   <211> 3651
   <212> DNA
   <213> Artificial Sequence
<220>
   <223> Description of Artificial Sequence: hybrid protoxin gene SN15
<400> 1
   atgaaactaa agaatcaaga taagcatcaa agtttttcta gcaatgcgaa agtagataaa 60
   atctctacgg attcactaaa aaatgaaaca gatatagaat tacaaaacat taatcatgaa 120
   gattgtttga aaatgtctga gtatgaaaat gtagagccgt ttgttagtgc atcaacaatt 180
   caaacaggta ttggtattgc gggtaaaata cttggtaccc taggcgttcc ttttgcagga 240
   caagtagcta gtctttatag ttttatctta ggtgagctat ggcctaaggg gaaaaatcaa 300
   tgggaaatct ttatggaaca tgtagaagag attattaatc aaaaaatatc aacttatgca 360
   agaaataaag cacttacaga cttgaaagga ttaggagatg ccttagctgt ctaccatgat 420
   tcgcttgaaa gttgggttgg aaatcgtaat aacacaaggg ctaggagtgt tgtcaagagc 480
   caatatatcg cattagaatt gatgttcgtt cagaaactac cttcttttgc agtgtctgga 540
   gaggaggtac cattattacc gatatatgcc caagctgcaa atttacattt gttgctatta 600
   agagatgcat ctatttttgg aaaagagtgg ggattatcat cttcagaaat ttcaacattt 660
   tataaccgtc aagtcgaacg agcaggagat tattcctacc attgtgtgaa atggtatagc 720
   acaggtctaa ataacttgag gggtacaaat gccgaaagtt gggtacgata taatcaattc 780
   cgtagagaca tgactttaat ggtactagat ttagtggcac tatttccaag ctatgataca 840
   caaatgtatc caattaaaac tacagcccaa cttacaagag aagtatatac agacgcaatt 900
   gggacagtac atccgcatcc aagttttaca agtacgactt ggtataataa taatgcacct 960
   tcgttctctg ccatagaggc tgctgttgtt cgaaacccgc atctactcga ttttctagaa 1020
   caagttacaa tttacagctt attaagtcga tggagtaaca ctcagtatat gaatatgtgg 1080
   ggaggacata aactagaatt ccgaacaata ggaggaacgt taaatatctc aacacaagga 1140
   tctactaata cttctattaa tcctgtaaca ttaccgttca cttctcgaga cgtctatagg 1200
   actgaatcat tggcagggct gaatctattt ttaactcaac ctgttaatgg agtacctagg 1260
   gttgattttc attggaaatt cgtcacacat ccgatcgcat ctgataattt ctattatcca 1320
   gggtatgctg gaattgggac gcaattacag gattcagaaa atgaattacc acctgaagca 1380
   acaggacagc caaattatga atcttatagt catagattat ctcatatagg actcatttca 1440
   gcatcacatg tgaaagcatc ggtatattct tggacgcatc gtagtgcgga ccgtacgaat 1500
   acgattggac caaatagaat cacccaaatc ccaatggtaa aagcatccga acttcctcaa 1560
   ggtaccactg ttgttagagg accaggattt actggtgggg atattcttcg aagaacgaat 1620
   actggtggat ttggaccgat aagagtaact gttaacggac cattaacaca aagatatcgt 1680
   ataggattcc gctatgcttc aactgtagat tttgatttct ttgtatcacg tggaggtact 1740
   actgtaaata attttagatt cctacgtaca atgaacagtg gagacgaact aaaatacgga 1800
   aattttgtga gacgtgcttt tactacacct tttactttta cacaaattca agatataatt 1860
   cgaacgtcta ttcaaggcct tagtggaaat ggggaagtgt atatagataa aattgaaatt 1920
   attccagtta ctgcaacctt cgaagcagaa tatgatttag aaagagcgca agaggcggtg 1980
   aatgctctgt ttactaatac gaatccaaga agattgaaaa cagatgtgac agattatcat 2040
   attgatcaag tatccaattt agtggattgt ttatcagatg aattttgtct ggatgaaaag 2100
   cgagaattgt ccgagaaagt caaacatgcg aagcgactca gtgatgagcg gaatttactt 2160
   caagatccaa acttcagagg gatcaataga caaccagacc gtggctggag aggaagtaca 2220
   gatattacca tccaaggagg agatgacgta ttcaaagaga attacgtcac actaccgggt 2280
   accgttgatg agtgctatcc aacgtattta tatcagaaaa tagatgagtc gaaattaaaa 2340
   gcttataccc gttatgaatt aagagggtat atcgaagata gtcaagactt agaaatctat 2400
   ttgatccgtt acaatgcaaa acacgaaata gtaaatgtgc caggcacggg ttccttatgg 2460
   ccgctttcag cccaaagtcc aatcggaaag tgtggagaac cgaatcgatg cgcgccacac 2520
   cttgaatgga atcctgatct agattgttcc tgcagagacg gggaaaaatg tgcacatcat 2580
   tcccatcatt tcaccttgga tattgatgtt ggatgtacag acttaaatga ggacttaggt 2640
   gtatgggtga tattcaagat taagacgcaa gatggccatg caagactagg gaatctagag 2700
   tttctcgaag agaaaccatt attaggggaa gcactagctc gtgtgaaaag agcggagaag 2760
   aagtggagag acaaacgaga gaaactgcag ttggaaacaa atattgttta taaagaggca 2820
   aaagaatctg tagatgcttt atttgtaaac tctcaatatg atagattaca agtggatacg 2880
   aacatcgcga tgattcatgc ggcagataaa cgcgttcata gaatccggga agcgtatctg 2940
   ccagagttgt ctgtgattcc aggtgtcaat gcggccattt tcgaagaatt agagggacgt 3000
   atttttacag cgtattcctt atatgatgcg agaaatgtca ttaaaaatgg cgatttcaat 3060
   aatggcttat tatgctggaa cgtgaaaggt catgtagatg tagaagagca aaacaaccac 3120
   cgttcggtcc ttgttatccc agaatgggag gcagaagtgt cacaagaggt tcgtgtctgt 3180
   ccaggtcgtg gctatatcct tcgtgtcaca gcatataaag agggatatgg agagggctgc 3240
   gtaacgatcc atgagatcga agacaataca gacgaactga aattcagcaa ctgtgtagaa 3300
   gaggaagtat atccaaacaa cacagtaacg tgtaataatt atactgggac tcaagaagaa 3360
   tatgagggta cgtacacttc tcgtaatcaa ggatatgacg aagcctatgg taataaccct 3420
   tccgtaccag ctgattacgc ttcagtctat gaagaaaaat cgtatacaga tggacgaaga 3480
   gagaatcctt gtgaatctaa cagaggctat ggggattaca caccactacc ggctggttat 3540
   gtaacaaagg atttagagta cttcccagag accgataagg tatggattga gatcggagaa 3600
   acagaaggaa cattcatcgt ggatagcgtg gaattactcc ttatggagga a          3651
<210> 2
   <211> 1217
   <212> PRT
   <213> Artificial Sequence
<220>
   <223> Description of Artificial Sequence: protein encoded by the gene SN15
<400> 2
   Met Lys Leu Lys Asn Gln Asp Lys His Gln Ser Phe Ser Ser Asn Ala
   1               5                   10                  15
   Lys Val Asp Lys Ile Ser Thr Asp Ser Leu Lys Asn Glu Thr Asp Ile
               20                  25                  30
   Glu Leu Gln Asn Ile Asn His Glu Asp Cys Leu Lys Met Ser Glu Tyr
           35                  40                  45
   Glu Asn Val Glu Pro Phe Val Ser Ala Ser Thr Ile Gln Thr Gly Ile
       50                  55                  60
   Gly Ile Ala Gly Lys Ile Leu Gly Thr Leu Gly Val Pro Phe Ala Gly
   65                  70                  75                  80
   Gln Val Ala Ser Leu Tyr Ser Phe Ile Leu Gly Glu Leu Trp Pro Lys
                   85                  90                  95
   Gly Lys Asn Gln Trp Glu Ile Phe Met Glu His Val Glu Glu Ile Ile
               100                 105                 110
   Asn Gln Lys Ile Ser Thr Tyr Ala Arg Asn Lys Ala Leu Thr Asp Leu
           115                 120                 125
   Lys Gly Leu Gly Asp Ala Leu Ala Val Tyr His Asp Ser Leu Glu Ser
       130                 135                 140
   Trp Val Gly Asn Arg Asn Asn Thr Arg Ala Arg Ser Val Val Lys Ser
   145                 150                 155                 160
   Gln Tyr Ile Ala Leu Glu Leu Met Phe Val Gln Lys Leu Pro Ser Phe
                   165                 170                 175
   Ala Val Ser Gly Glu Glu Val Pro Leu Leu Pro Ile Tyr Ala Gln Ala
               180                 185                 190
   Ala Asn Leu His Leu Leu Leu Leu Arg Asp Ala Ser Ile Phe Gly Lys
           195                 200                 205
   Glu Trp Gly Leu Ser Ser Ser Glu Ile Ser Thr Phe Tyr Asn Arg Gln
       210                 215                 220
   Val Glu Arg Ala Gly Asp Tyr Ser Tyr His Cys Val Lys Trp Tyr Ser
   225                 230                 235                 240
   Thr Gly Leu Asn Asn Leu Arg Gly Thr Asn Ala Glu Ser Trp Val Arg
   245 250 255
   Tyr Asn Gln Phe Arg Arg Asp Met Thr Leu Met Val Leu Asp Leu Val
   260 265 270
   Ala Leu Phe Pro Ser Tyr Asp Thr Gln Met Tyr Pro Ile Lys Thr Thr
   275 280 285
   Ala Gln Leu Thr Arg Glu Val Tyr Thr Asp Ala Ile Gly Thr Val His
   290 295 300
   Pro His Pro Ser Phe Thr Ser Thr Thr Trp Tyr Asn Asn Asn Ala Pro
   305 310 315 320
   Ser Phe Ser Ala Ile Glu Ala Ala Val Val Arg Asn Pro His Leu Leu
   325 330 335
   Asp Phe Leu Glu Gln Val Thr Ile Tyr Ser Leu Leu Ser Arg Trp Ser
   340 345 350
   Asn Thr Gln Tyr Met Asn Met Trp Gly Gly His Lys Leu Glu Phe Arg
   355 360 365
   Thr Ile Gly Gly Thr Leu Asn Ile Ser Thr Gln Gly Ser Thr Asn Thr
   370 375 380
   Ser Ile Asn Pro Val Thr Leu Pro Phe Thr Ser Arg Asp Val Tyr Arg
   385 390 395 400
   Thr Glu Ser Leu Ala Gly Leu Asn Leu Phe Leu Thr Gln Pro Val Asn
   405 410 415
   Gly Val Pro Arg Val Asp Phe His Trp Lys Phe Val Thr His Pro Ile
   420 425 430
   Ala Ser Asp Asn Phe Tyr Tyr Pro Gly Tyr Ala Gly Ile Gly Thr Gln
   435 440 445
   Leu Gln Asp Ser Glu Asn Glu Leu Pro Pro Glu Ala Thr Gly Gln Pro
   450 455 460
   Asn Tyr Glu Ser Tyr Ser His Arg Leu Ser His Ile Gly Leu Ile Ser
   465 470 475 480
   Ala Ser His Val Lys Ala Ser Val Tyr Ser Trp Thr His Arg Ser Ala
   485 490 495
   Asp Arg Thr Asn Thr Ile Gly Pro Asn Arg Ile Thr Gln Ile Pro Met
   500 505 510
   Val Lys Ala Ser Glu Leu Pro Gln Gly Thr Thr Val Val Arg Gly Pro
   515 520 525
   Gly Phe Thr Gly Gly Asp Ile Leu Arg Arg Thr Asn Thr Gly Gly Phe
   530 535 540
   Gly Pro Ile Arg Val Thr Val Asn Gly Pro Leu Thr Gln Arg Tyr Arg
   545 550 555 560
   Ile Gly Phe Arg Tyr Ala Ser Thr Val Asp Phe Asp Phe Phe Val Ser
   565 570 575
   Arg Gly Gly Thr Thr Val Asn Asn Phe Arg Phe Leu Arg Thr Met Asn
   580 585 590
   Ser Gly Asp Glu Leu Lys Tyr Gly Asn Phe Val Arg Arg Ala Phe Thr
   595 600 605
   Thr Pro Phe Thr Phe Thr Gln Ile Gln Asp Ile Ile Arg Thr Ser Ile
   610 615 620
   Gln Gly Leu Ser Gly Asn Gly Glu Val Tyr Ile Asp Lys Ile Glu Ile
   625 630 635 640
   Ile Pro Val Thr Ala Thr Phe Glu Ala Glu Tyr Asp Leu Glu Arg Ala
   645 650 655
   Gln Glu Ala Val Asn Ala Leu Phe Thr Asn Thr Asn Pro Arg Arg Leu
   660 665 670
   Lys Thr Asp Val Thr Asp Tyr His Ile Asp Gln Val Ser Asn Leu Val
   675 680 685
   Asp Cys Leu Ser Asp Glu Phe Cys Leu Asp Glu Lys Arg Glu Leu Ser
   690 695 700
   Glu Lys Val Lys His Ala Lys Arg Leu Ser Asp Glu Arg Asn Leu Leu
   705 710 715 720
   Gln Asp Pro Asn Phe Arg Gly Ile Asn Arg Gln Pro Asp Arg Gly Trp
   725 730 735
   Arg Gly Ser Thr Asp Ile Thr Ile Gln Gly Gly Asp Asp Val Phe Lys
   740 745 750
   Glu Asn Tyr Val Thr Leu Pro Gly Thr Val Asp Glu Cys Tyr Pro Thr
   755 760 765
   Tyr Leu Tyr Gln Lys Ile Asp Glu Ser Lys Leu Lys Ala Tyr Thr Arg
   770 775 780
   Tyr Glu Leu Arg Gly Tyr Ile Glu Asp Ser Gln Asp Leu Glu Ile Tyr
   785 790 795 800
   Leu Ile Arg Tyr Asn Ala Lys His Glu Ile Val Asn Val Pro Gly Thr
   805 810 815
   Gly Ser Leu Trp Pro Leu Ser Ala Gln Ser Pro Ile Gly Lys Cys Gly
   820 825 830
   Glu Pro Asn Arg Cys Ala Pro His Leu Glu Trp Asn Pro Asp Leu Asp
   835 840 845
   Cys Ser Cys Arg Asp Gly Glu Lys Cys Ala His His Ser His His Phe
   850 855 860
   Thr Leu Asp Ile Asp Val Gly Cys Thr Asp Leu Asn Glu Asp Leu Gly
   865 870 875 880
   Val Trp Val Ile Phe Lys Ile Lys Thr Gln Asp Gly His Ala Arg Leu
   885 890 895
   Gly Asn Leu Glu Phe Leu Glu Glu Lys Pro Leu Leu Gly Glu Ala Leu
   900 905 910
   Ala Arg Val Lys Arg Ala Glu Lys Lys Trp Arg Asp Lys Arg Glu Lys
   915 920 925
   Leu Gln Leu Glu Thr Asn Ile Val Tyr Lys Glu Ala Lys Glu Ser Val
   930 935 940
   Asp Ala Leu Phe Val Asn Ser Gln Tyr Asp Arg Leu Gln Val Asp Thr
   945 950 955 960
   Asn Ile Ala Met Ile His Ala Ala Asp Lys Arg Val His Arg Ile Arg
   965 970 975
   Glu Ala Tyr Leu Pro Glu Leu Ser Val Ile Pro Gly Val Asn Ala Ala
   980 985 990
   Ile Phe Glu Glu Leu Glu Gly Arg Ile Phe Thr Ala Tyr Ser Leu Tyr
   995 1000 1005
   Asp Ala Arg Asn Val Ile Lys Asn Gly Asp Phe Asn Asn Gly Leu Leu
   1010 1015 1020
   Cys Trp Asn Val Lys Gly His Val Asp Val Glu Glu Gln Asn Asn His
   1025 1030 1035 1040
   Arg Ser Val Leu Val Ile Pro Glu Trp Glu Ala Glu Val Ser Gln Glu
   1045 1050 1055
   Val Arg Val Cys Pro Gly Arg Gly Tyr Ile Leu Arg Val Thr Ala Tyr
   1060 1065 1070
   Lys Glu Gly Tyr Gly Glu Gly Cys Val Thr Ile His Glu Ile Glu Asp
   1075 1080 1085
   Asn Thr Asp Glu Leu Lys Phe Ser Asn Cys Val Glu Glu Glu Val Tyr
   1090 1095 1100
   Pro Asn Asn Thr Val Thr Cys Asn Asn Tyr Thr Gly Thr Gln Glu Glu
   1105 1110 1115 1120
   Tyr Glu Gly Thr Tyr Thr Ser Arg Asn Gln Gly Tyr Asp Glu Ala Tyr
   1125 1130 1135
   Gly Asn Asn Pro Ser Val Pro Ala Asp Tyr Ala Ser Val Tyr Glu Glu
   1140 1145 1150
   Lys Ser Tyr Thr Asp Gly Arg Arg Glu Asn Pro Cys Glu Ser Asn Arg
   1155 1160 1165
   Gly Tyr Gly Asp Tyr Thr Pro Leu Pro Ala Gly Tyr Val Thr Lys Asp
   1170 1175 1180
   Leu Glu Tyr Phe Pro Glu Thr Asp Lys Val Trp Ile Glu Ile Gly Glu
   1185 1190 1195 1200
   Thr Glu Gly Thr Phe Ile Val Asp Ser Val Glu Leu Leu Leu Met Glu
   1205 1210 1215
   Glu
<210> 3
   <211> 3624
   <212> DNA
   <213> Artificial Sequence
<220>
   <223> Description of Artificial Sequence: hybrid protoxin gene SN19
<400> 3
   atggcttcaa ataggaaaaa tgagaatgaa attataaatg ctgtatcgaa tcattccgca 60
   caaatggatc tattaccaga tgctcgtatt gaggatagct tgtgtatagc cgaggggaac 120
   aatatcgatc catttgttag cgcatcaaca gtccaaacgg gtattaacat agctggtaga 180
   atactaggcg tattgggcgt accgtttgct ggacaactag ctagttttta tagttttctt 240
   gttggtgaat tatggccccg cggcagagat cagtgggaaa ttttcctaga acatgtcgaa 300
   caacttataa atcaacaaat aacagaaaat gctaggaata cggctcttgc tcgattacaa 360
   ggtttaggag attccttcag agcctatcaa cagtcacttg aagattggct agaaaaccgt 420
   gatgatgcaa gaacgagaag tgttctttat acccaatata tagctttaga acttgatttt 480
   cttaatgcga tgccgctttt cgcaattaga aaccaagaag ttccattatt gatggtatat 540
   gctcaagctg caaatttaca cctattatta ttgagagatg cctctctttt tggtagtgaa 600
   tttgggctta catcgcagga aattcaacgc tattatgagc gccaagtgga acgaacgaga 660
   gattattccg actattgcgt agaatggtat aatacaggtc taaatagctt gagagggaca 720
   aatgccgcaa gttgggtacg gtataatcaa ttccgtagag atctaacgtt aggagtatta 780
   gatctagtgg cactattccc aagctatgac actcgcactt atccaataaa tacgagtgct 840
   cagttaacaa gagaagttta tacagacgca attgggacag tacatccgca tccaagtttt 900
   acaagtacga cttggtataa taataatgca ccttcgttct ctgccataga ggctgctgtt 960
   gttcgaaacc cgcatctact cgattttcta gaacaagtta caatttacag cttattaagt 1020
   cgatggagta acactcagta tatgaatatg tggggaggac ataaactaga attccgaaca 1080
   ataggaggaa cgttaaatat ctcaacacaa ggatctacta atacttctat taatcctgta 1140
   acattaccgt tcacttctcg agacgtctat aggactgaat cattggcagg gctgaatcta 1200
   tttttaactc aacctgttaa tggagtacct agggttgatt ttcattggaa attcgtcaca 1260
   catccgatcg catctgataa tttctattat ccagggtatg ctggaattgg gacgcaatta 1320
   caggattcag aaaatgaatt accacctgaa gcaacaggac agccaaatta tgaatcttat 1380
   agtcatagat tatctcatat aggactcatt tcagcatcac atgtgaaagc atcggtatat 1440
   tcttggacgc atcgtagtgc ggaccgtacg aatacgattg gaccaaatag aatcacccaa 1500
   atcccaatgg taaaagcatc cgaacttcct caaggtacca ctgttgttag aggaccagga 1560
   tttactggtg gggatattct tcgaagaacg aatactggtg gatttggacc gataagagta 1620
   actgttaacg gaccattaac acaaagatat cgtataggat tccgctatgc ttcaactgta 1680
   gattttgatt tctttgtatc acgtggaggt actactgtaa ataattttag attcctacgt 1740
   acaatgaaca gtggagacga actaaaatac ggaaattttg tgagacgtgc ttttactaca 1800
   ccttttactt ttacacaaat tcaagatata attcgaacgt ctattcaagg ccttagtgga 1860
   aatggggaag tgtatataga taaaattgaa attattccag ttactgcaac cttcgaagca 1920
   gaatatgatt tagaaagagc gcaagaggcg gtgaatgctc tgtttactaa tacgaatcca 1980
   agaagattga aaacagatgt gacagattat catattgatc aagtatccaa tttagtggat 2040
   tgtttatcag atgaattttg tctggatgaa aagcgagaat tgtccgagaa agtcaaacat 2100
   gcgaagcgac tcagtgatga gcggaattta cttcaagatc caaacttcag agggatcaat 2160
   agacaaccag accgtggctg gagaggaagt acagatatta ccatccaagg aggagatgac 2220
   gtattcaaag agaattacgt cacactaccg ggtaccgttg atgagtgcta tccaacgtat 2280
   ttatatcaga aaatagatga gtcgaaatta aaagcttata cccgttatga attaagaggg 2340
   tatatcgaag atagtcaaga cttagaaatc tatttgatcc gttacaatgc aaaacacgaa 2400
   atagtaaatg tgccaggcac gggttcctta tggccgcttt cagcccaaag tccaatcgga 2460
   aagtgtggag aaccgaatcg atgcgcgcca caccttgaat ggaatcctga tctagattgt 2520
   tcctgcagag acggggaaaa atgtgcacat cattcccatc atttcacctt ggatattgat 2580
   gttggatgta cagacttaaa tgaggactta ggtgtatggg tgatattcaa gattaagacg 2640
   caagatggcc atgcaagact agggaatcta gagtttctcg aagagaaacc attattaggg 2700
   gaagcactag ctcgtgtgaa aagagcggag aagaagtgga gagacaaacg agagaaactg 2760
   cagttggaaa caaatattgt ttataaagag gcaaaagaat ctgtagatgc tttatttgta 2820
   aactctcaat atgatagatt acaagtggat acgaacatcg cgatgattca tgcggcagat 2880
   aaacgcgttc atagaatccg ggaagcgtat ctgccagagt tgtctgtgat tccaggtgtc 2940
   aatgcggcca ttttcgaaga attagaggga cgtattttta cagcgtattc cttatatgat 3000
   gcgagaaatg tcattaaaaa tggcgatttc aataatggct tattatgctg gaacgtgaaa 3060
   ggtcatgtag atgtagaaga gcaaaacaac caccgttcgg tccttgttat cccagaatgg 3120
   gaggcagaag tgtcacaaga ggttcgtgtc tgtccaggtc gtggctatat ccttcgtgtc 3180
   acagcatata aagagggata tggagagggc tgcgtaacga tccatgagat cgaagacaat 3240
   acagacgaac tgaaattcag caactgtgta gaagaggaag tatatccaaa caacacagta 3300
   acgtgtaata attatactgg gactcaagaa gaatatgagg gtacgtacac ttctcgtaat 3360
   caaggatatg acgaagccta tggtaataac ccttccgtac cagctgatta cgcttcagtc 3420
   tatgaagaaa aatcgtatac agatggacga agagagaatc cttgtgaatc taacagaggc 3480
   tatggggatt acacaccact accggctggt tatgtaacaa aggatttaga gtacttccca 3540
   gagaccgata aggtatggat tgagatcgga gaaacagaag gaacattcat cgtggatagc 3600
   gtggaattac tccttatgga ggaa 3624
<210> 4
   <211> 1208
   <212> PRT
   <213> Artificial Sequence
<220>
   <223> Description of Artificial Sequence: protein encoded by the gene SN19
<400> 4
   Met Ala Ser Asn Arg Lys Asn Glu Asn Glu Ile Ile Asn Ala Val Ser
   1               5                   10                  15
   Asn His Ser Ala Gln Met Asp Leu Leu Pro Asp Ala Arg Ile Glu Asp
               20                  25                  30
   Ser Leu Cys Ile Ala Glu Gly Asn Asn Ile Asp Pro Phe Val Ser Ala
           35                  40                  45
   Ser Thr Val Gln Thr Gly Ile Asn Ile Ala Gly Arg Ile Leu Gly Val
       50                  55                  60
   Leu Gly Val Pro Phe Ala Gly Gln Leu Ala Ser Phe Tyr Ser Phe Leu
   65                  70                  75                  80
   Val Gly Glu Leu Trp Pro Arg Gly Arg Asp Gln Trp Glu Ile Phe Leu
                   85                  90                  95
   Glu His Val Glu Gln Leu Ile Asn Gln Gln Ile Thr Glu Asn Ala Arg
   100 105 110
   Asn Thr Ala Leu Ala Arg Leu Gln Gly Leu Gly Asp Ser Phe Arg Ala
   115 120 125
   Tyr Gln Gln Ser Leu Glu Asp Trp Leu Glu Asn Arg Asp Asp Ala Arg
   130 135 140
   Thr Arg Ser Val Leu Tyr Thr Gln Tyr Ile Ala Leu Glu Leu Asp Phe
   145 150 155 160
   Leu Asn Ala Met Pro Leu Phe Ala Ile Arg Asn Gln Glu Val Pro Leu
   165 170 175
   Leu Met Val Tyr Ala Gln Ala Ala Asn Leu His Leu Leu Leu Leu Arg
   180 185 190
   Asp Ala Ser Leu Phe Gly Ser Glu Phe Gly Leu Thr Ser Gln Glu Ile
   195 200 205
   Gln Arg Tyr Tyr Glu Arg Gln Val Glu Arg Thr Arg Asp Tyr Ser Asp
   210 215 220
   Tyr Cys Val Glu Trp Tyr Asn Thr Gly Leu Asn Ser Leu Arg Gly Thr
   225 230 235 240
   Asn Ala Ala Ser Trp Val Arg Tyr Asn Gln Phe Arg Arg Asp Leu Thr
   245 250 255
   Leu Gly Val Leu Asp Leu Val Ala Leu Phe Pro Ser Tyr Asp Thr Arg
   260 265 270
   Thr Tyr Pro Ile Asn Thr Ser Ala Gln Leu Thr Arg Glu Val Tyr Thr
   275 280 285
   Asp Ala Ile Gly Thr Val His Pro His Pro Ser Phe Thr Ser Thr Thr
   290 295 300
   Trp Tyr Asn Asn Asn Ala Pro Ser Phe Ser Ala Ile Glu Ala Ala Val
   305 310 315 320
   Val Arg Asn Pro His Leu Leu Asp Phe Leu Glu Gln Val Thr Ile Tyr
   325 330 335
   Ser Leu Leu Ser Arg Trp Ser Asn Thr Gln Tyr Met Asn Met Trp Gly
   340 345 350
   Gly His Lys Leu Glu Phe Arg Thr Ile Gly Gly Thr Leu Asn Ile Ser
   355 360 365
   Thr Gln Gly Ser Thr Asn Thr Ser Ile Asn Pro Val Thr Leu Pro Phe
   370 375 380
   Thr Ser Arg Asp Val Tyr Arg Thr Glu Ser Leu Ala Gly Leu Asn Leu
   385 390 395 400
   Phe Leu Thr Gln Pro Val Asn Gly Val Pro Arg Val Asp Phe His Trp
   405 410 415
   Lys Phe Val Thr His Pro Ile Ala Ser Asp Asn Phe Tyr Tyr Pro Gly
   420 425 430
   Tyr Ala Gly Ile Gly Thr Gln Leu Gln Asp Ser Glu Asn Glu Leu Pro
   435 440 445
   Pro Glu Ala Thr Gly Gln Pro Asn Tyr Glu Ser Tyr Ser His Arg Leu
   450 455 460
   Ser His Ile Gly Leu Ile Ser Ala Ser His Val Lys Ala Ser Val Tyr
   465 470 475 480
   Ser Trp Thr His Arg Ser Ala Asp Arg Thr Asn Thr Ile Gly Pro Asn
   485 490 495
   Arg Ile Thr Gln Ile Pro Met Val Lys Ala Ser Glu Leu Pro Gln Gly
   500 505 510
   Thr Thr Val Val Arg Gly Pro Gly Phe Thr Gly Gly Asp Ile Leu Arg
   515 520 525
   Arg Thr Asn Thr Gly Gly Phe Gly Pro Ile Arg Val Thr Val Asn Gly
   530 535 540
   Pro Leu Thr Gln Arg Tyr Arg Ile Gly Phe Arg Tyr Ala Ser Thr Val
   545 550 555 560
   Asp Phe Asp Phe Phe Val Ser Arg Gly Gly Thr Thr Val Asn Asn Phe
   565 570 575
   Arg Phe Leu Arg Thr Met Asn Ser Gly Asp Glu Leu Lys Tyr Gly Asn
   580 585 590
   Phe Val Arg Arg Ala Phe Thr Thr Pro Phe Thr Phe Thr Gln Ile Gln
   595 600 605
   Asp Ile Ile Arg Thr Ser Ile Gln Gly Leu Ser Gly Asn Gly Glu Val
   610 615 620
   Tyr Ile Asp Lys Ile Glu Ile Ile Pro Val Thr Ala Thr Phe Glu Ala
   625 630 635 640
   Glu Tyr Asp Leu Glu Arg Ala Gln Glu Ala Val Asn Ala Leu Phe Thr
   645 650 655
   Asn Thr Asn Pro Arg Arg Leu Lys Thr Asp Val Thr Asp Tyr His Ile
   660 665 670
   Asp Gln Val Ser Asn Leu Val Asp Cys Leu Ser Asp Glu Phe Cys Leu
   675 680 685
   Asp Glu Lys Arg Glu Leu Ser Glu Lys Val Lys His Ala Lys Arg Leu
   690 695 700
   Ser Asp Glu Arg Asn Leu Leu Gln Asp Pro Asn Phe Arg Gly Ile Asn
   705 710 715 720
   Arg Gln Pro Asp Arg Gly Trp Arg Gly Ser Thr Asp Ile Thr Ile Gln
   725 730 735
   Gly Gly Asp Asp Val Phe Lys Glu Asn Tyr Val Thr Leu Pro Gly Thr
   740 745 750
   Val Asp Glu Cys Tyr Pro Thr Tyr Leu Tyr Gln Lys Ile Asp Glu Ser
   755 760 765
   Lys Leu Lys Ala Tyr Thr Arg Tyr Glu Leu Arg Gly Tyr Ile Glu Asp
   770 775 780
   Ser Gln Asp Leu Glu Ile Tyr Leu Ile Arg Tyr Asn Ala Lys His Glu
   785 790 795 800
   Ile Val Asn Val Pro Gly Thr Gly Ser Leu Trp Pro Leu Ser Ala Gln
   805 810 815
   Ser Pro Ile Gly Lys Cys Gly Glu Pro Asn Arg Cys Ala Pro His Leu
   820 825 830
   Glu Trp Asn Pro Asp Leu Asp Cys Ser Cys Arg Asp Gly Glu Lys Cys
   835 840 845
   Ala His His Ser His His Phe Thr Leu Asp Ile Asp Val Gly Cys Thr
   850 855 860
   Asp Leu Asn Glu Asp Leu Gly Val Trp Val Ile Phe Lys Ile Lys Thr
   865                 870                 875                 880
   Gln Asp Gly His Ala Arg Leu Gly Asn Leu Glu Phe Leu Glu Glu Lys
                   885                 890                 895
   Pro Leu Leu Gly Glu Ala Leu Ala Arg Val Lys Arg Ala Glu Lys Lys
               900                 905                 910
   Trp Arg Asp Lys Arg Glu Lys Leu Gln Leu Glu Thr Asn Ile Val Tyr
           915                 920                 925
   Lys Glu Ala Lys Glu Ser Val Asp Ala Leu Phe Val Asn Ser Gln Tyr
       930                 935                 940
   Asp Arg Leu Gln Val Asp Thr Asn Ile Ala Met Ile His Ala Ala Asp
   945                 950                 955                 960
   Lys Arg Val His Arg Ile Arg Glu Ala Tyr Leu Pro Glu Leu Ser Val
                   965                 970                 975
   Ile Pro Gly Val Asn Ala Ala Ile Phe Glu Glu Leu Glu Gly Arg Ile
               980                 985                 990
   Phe Thr Ala Tyr Ser Leu Tyr Asp Ala Arg Asn Val Ile Lys Asn Gly
           995                 1000                1005
   Asp Phe Asn Asn Gly Leu Leu Cys Trp Asn Val Lys Gly His Val Asp
       1010                1015                1020
   Val Glu Glu Gln Asn Asn His Arg Ser Val Leu Val Ile Pro Glu Trp
   1025                1030                1035                1040
   Glu Ala Glu Val Ser Gln Glu Val Arg Val Cys Pro Gly Arg Gly Tyr
                   1045                1050                1055
   Ile Leu Arg Val Thr Ala Tyr Lys Glu Gly Tyr Gly Glu Gly Cys Val
               1060                1065                1070
   Thr Ile His Glu Ile Glu Asp Asn Thr Asp Glu Leu Lys Phe Ser Asn
           1075                1080                1085
   Cys Val Glu Glu Glu Val Tyr Pro Asn Asn Thr Val Thr Cys Asn Asn
       1090                1095                1100
   Tyr Thr Gly Thr Gln Glu Glu Tyr Glu Gly Thr Tyr Thr Ser Arg Asn
   1105                1110                1115                1120
   Gln Gly Tyr Asp Glu Ala Tyr Gly Asn Asn Pro Ser Val Pro Ala Asp
   1125 1130 1135
   Tyr Ala Ser Val Tyr Glu Glu Lys Ser Tyr Thr Asp Gly Arg Arg Glu
   1140 1145 1150
   Asn Pro Cys Glu Ser Asn Arg Gly Tyr Gly Asp Tyr Thr Pro Leu Pro
   1155 1160 1165
   Ala Gly Tyr Val Thr Lys Asp Leu Glu Tyr Phe Pro Glu Thr Asp Lys
   1170 1175 1180
   Val Trp Ile Glu Ile Gly Glu Thr Glu Gly Thr Phe Ile Val Asp Ser
   1185 1190 1195 1200
   Val Glu Leu Leu Leu Met Glu Glu
   1205
<210> 5
   <211> 3627
   <212> DNA
   <213> Artificial Sequence
<220>
   <223> Description of Artificial Sequence: modified cry1Ba gene
<400> 5
   atgacttcaa ataggaaaaa tgagaatgaa attataaatg ctgtatcgaa tcattccgca 60
   caaatggatc tattaccaga tgctcgtatt gaggatagct tgtgtatagc cgaggggaac 120
   aatatcgatc catttgttag cgcatcaaca gtccaaacgg gtattaacat agctggtaga 180
   atactaggcg tattgggcgt accgtttgct ggacaactag ctagttttta tagttttctt 240
   gttggtgaat tatggccccg cggcagagat cagtgggaaa ttttcctaga acatgtcgaa 300
   caacttataa atcaacaaat aacagaaaat gctaggaata cggctcttgc tcgattacaa 360
   ggtttaggag attccttcag agcctatcaa cagtcacttg aagattggct agaaaaccgt 420
   gatgatgcaa gaacgagaag tgttctttat acccaatata tagctttaga acttgatttt 480
   cttaatgcga tgccgctttt cgcaattaga aaccaagaag ttccattatt gatggtatat 540
   gctcaagctg caaatttaca cctattatta ttgagagatg cctctctttt tggtagtgaa 600
   tttgggctta catcgcagga aattcaacgc tattatgagc gccaagtgga acgaacgaga 660
   gattattccg actattgcgt agaatggtat aatacaggtc taaatagctt gagagggaca 720
   aatgccgcaa gttgggtacg gtataatcaa ttccgtagag atctaacgtt aggagtatta 780
   gatctagtgg cactattccc aagctatgac actcgcactt atccaataaa tacgagtgct 840
   cagttaacaa gagaagttta tacagacgca attggagcaa caggggtaaa tatggcaagt 900
   atgaattggt ataataataa tgcaccttcg ttctctgcca tagaggctgc ggctatccga 960
   agcccgcatc tacttgattt tctagaacaa cttacaattt ttagcgcttc atcacgatgg 1020
   agtaatacta ggcatatgac ttattggcgg gggcacacga ttcaatctcg gccaatagga 1080
   ggcggattaa atacctcaac gcatggggct accaatactt ctattaatcc tgtaacatta 1140
   cggttcgcat ctcgagacgt ttataggact gaatcatatg caggagtgct tctatgggga 1200
   atttaccttg aacctattca tggtgtccct actgttaggt ttaattttac gaaccctcag 1260
   aatatttctg atagaggtac cgctaactat agtcaacctt atgagtcacc tgggcttcaa 1320
   ttaaaagatt cagaaactga attaccacca gaaacaacag aacgaccaaa ttatgaatct 1380
   tacagtcaca ggttatctca tataggtata attttacaat ccagggtgaa tgtaccggta 1440
   tattcttgga cgcatcgtag tgcggaccgt acgaatacga ttggaccaaa tagaatcacc 1500
   caaatcccaa tggtaaaagc atccgaactt cctcaaggta ccactgttgt tagaggacca 1560
   ggatttactg gtggggatat tcttcgaaga acgaatactg gtggatttgg accgataaga 1620
   gtaactgtta acggaccatt aacacaaaga tatcgtatag gattccgcta tgcttcaact 1680
   gtagattttg atttctttgt atcacgtgga ggtactactg taaataattt tagattccta 1740
   cgtacaatga acagtggaga cgaactaaaa tacggaaatt ttgtgagacg tgcttttact 1800
   acacctttta cttttacaca aattcaagat ataattcgaa cgtctattca aggccttagt 1860
   ggaaatgggg aagtgtatat agataaaatt gaaattattc cagttactgc aaccttcgaa 1920
   gcagaatatg atttagaaag agcgcaagag gcggtgaatg ctctgtttac taatacgaat 1980
   ccaagaagat tgaaaacaga tgtgacagat tatcatattg atcaagtatc caatttagtg 2040
   gattgtttat cagatgaatt ttgtctggat gaaaagcgag aattgtccga gaaagtcaaa 2100
   catgcgaagc gactcagtga tgagcggaat ttacttcaag atccaaactt cagagggatc 2160
   aatagacaac cagaccgtgg ctggagagga agtacagata ttaccatcca aggaggagat 2220
   gacgtattca aagagaatta cgtcacacta ccgggtaccg ttgatgagtg ctatccaacg 2280
   tatttatatc agaaaataga tgagtcgaaa ttaaaagctt atacccgtta tgaattaaga 2340
   gggtatatcg aagatagtca agacttagaa atctatttga tccgttacaa tgcaaaacac 2400
   gaaatagtaa atgtgccagg cacgggttcc ttatggccgc tttcagccca aagtccaatc 2460
   ggaaagtgtg gagaaccgaa tcgatgcgcg ccacaccttg aatggaatcc tgatctagat 2520
   tgttcctgca gagacgggga aaaatgtgca catcattccc atcatttcac cttggatatt 2580
   gatgttggat gtacagactt aaatgaggac ttaggtgtat gggtgatatt caagattaag 2640
   acgcaagatg gccatgcaag actagggaat ctagagtttc tcgaagagaa accattatta 2700
   ggggaagcac tagctcgtgt gaaaagagcg gagaagaagt ggagagacaa acgagagaaa 2760
   ctgcagttgg aaacaaatat tgtttataaa gaggcaaaag aatctgtaga tgctttattt 2820
   gtaaactctc aatatgatag attacaagtg gatacgaaca tcgcgatgat tcatgcggca 2880
   gataaacgcg ttcatagaat ccgggaagcg tatctgccag agttgtctgt gattccaggt 2940
   gtcaatgcgg ccattttcga agaattagag ggacgtattt ttacagcgta ttccttatat 3000
   gatgcgagaa atgtcattaa aaatggcgat ttcaataatg gcttattatg ctggaacgtg 3060
   aaaggtcatg tagatgtaga agagcaaaac aaccaccgtt cggtccttgt tatcccagaa 3120
   tgggaggcag aagtgtcaca agaggttcgt gtctgtccag gtcgtggcta tatccttcgt 3180
   gtcacagcat ataaagaggg atatggagag ggctgcgtaa cgatccatga gatcgaagac 3240
   aatacagacg aactgaaatt cagcaactgt gtagaagagg aagtatatcc aaacaacaca 3300
   gtaacgtgta ataattatac tgggactcaa gaagaatatg agggtacgta cacttctcgt 3360
   aatcaaggat atgacgaagc ctatggtaat aacccttccg taccagctga ttacgcttca 3420
   gtctatgaag aaaaatcgta tacagatgga cgaagagaga atccttgtga atctaacaga 3480
   ggctatgggg attacacacc actaccggct ggttatgtaa caaaggattt agagtacttc 3540
   ccagagaccg ataaggtatg gattgagatc ggagaaacag aaggaacatt catcgtggat 3600
   agcgtggaat tactccttat ggaggaa 3627
<210> 6
   <211> 1209
   <212> PRT
<213> Artificial Sequence
<220>
   <223> Description of Artificial Sequence: protein encoded by the modified cry1Ba gene
<400> 6
   Met Thr Ser Asn Arg Lys Asn Glu Asn Glu Ile Ile Asn Ala Val Ser
   1 5 10 15
   Asn His Ser Ala Gln Met Asp Leu Leu Pro Asp Ala Arg Ile Glu Asp
   20 25 30
   Ser Leu Cys Ile Ala Glu Gly Asn Asn Ile Asp Pro Phe Val Ser Ala
   35 40 45
   Ser Thr Val Gln Thr Gly Ile Asn Ile Ala Gly Arg Ile Leu Gly Val
   50 55 60
   Leu Gly Val Pro Phe Ala Gly Gln Leu Ala Ser Phe Tyr Ser Phe Leu
   65 70 75 80
   Val Gly Glu Leu Trp Pro Arg Gly Arg Asp Gln Trp Glu Ile Phe Leu
   85 90 95
   Glu His Val Glu Gln Leu Ile Asn Gln Gln Ile Thr Glu Asn Ala Arg
   100 105 110
   Asn Thr Ala Leu Ala Arg Leu Gln Gly Leu Gly Asp Ser Phe Arg Ala
   115 120 125
   Tyr Gln Gln Ser Leu Glu Asp Trp Leu Glu Asn Arg Asp Asp Ala Arg
   130 135 140
   Thr Arg Ser Val Leu Tyr Thr Gln Tyr Ile Ala Leu Glu Leu Asp Phe
   145 150 155 160
   Leu Asn Ala Met Pro Leu Phe Ala Ile Arg Asn Gln Glu Val Pro Leu
   165 170 175
   Leu Met Val Tyr Ala Gln Ala Ala Asn Leu His Leu Leu Leu Leu Arg
   180 185 190
   Asp Ala Ser Leu Phe Gly Ser Glu Phe Gly Leu Thr Ser Gln Glu Ile
   195 200 205
   Gln Arg Tyr Tyr Glu Arg Gln Val Glu Arg Thr Arg Asp Tyr Ser Asp
   210 215 220
   Tyr Cys Val Glu Trp Tyr Asn Thr Gly Leu Asn Ser Leu Arg Gly Thr
   225 230 235 240
   Asn Ala Ala Ser Trp Val Arg Tyr Asn Gln Phe Arg Arg Asp Leu Thr
   245 250 255
   Leu Gly Val Leu Asp Leu Val Ala Leu Phe Pro Ser Tyr Asp Thr Arg
   260 265 270
   Thr Tyr Pro Ile Asn Thr Ser Ala Gln Leu Thr Arg Glu Val Tyr Thr
   275 280 285
   Asp Ala Ile Gly Ala Thr Gly Val Asn Met Ala Ser Met Asn Trp Tyr
   290 295 300
   Asn Asn Asn Ala Pro Ser Phe Ser Ala Ile Glu Ala Ala Ala Ile Arg
   305 310 315 320
   Ser Pro His Leu Leu Asp Phe Leu Glu Gln Leu Thr Ile Phe Ser Ala
   325 330 335
   Ser Ser Arg Trp Ser Asn Thr Arg His Met Thr Tyr Trp Arg Gly His
   340 345 350
   Thr Ile Gln Ser Arg Pro Ile Gly Gly Gly Leu Asn Thr Ser Thr His
   355 360 365
   Gly Ala Thr Asn Thr Ser Ile Asn Pro Val Thr Leu Arg Phe Ala Ser
   370 375 380
   Arg Asp Val Tyr Arg Thr Glu Ser Tyr Ala Gly Val Leu Leu Trp Gly
   385 390 395 400
   Ile Tyr Leu Glu Pro Ile His Gly Val Pro Thr Val Arg Phe Asn Phe
   405 410 415
   Thr Asn Pro Gln Asn Ile Ser Asp Arg Gly Thr Ala Asn Tyr Ser Gln
   420 425 430
   Pro Tyr Glu Ser Pro Gly Leu Gln Leu Lys Asp Ser Glu Thr Glu Leu
   435 440 445
   Pro Pro Glu Thr Thr Glu Arg Pro Asn Tyr Glu Ser Tyr Ser His Arg
   450 455 460
   Leu Ser His Ile Gly Ile Ile Leu Gln Ser Arg Val Asn Val Pro Val
   465 470 475 480
   Tyr Ser Trp Thr His Arg Ser Ala Asp Arg Thr Asn Thr Ile Gly Pro
                   485                 490                 495
   Asn Arg Ile Thr Gln Ile Pro Met Val Lys Ala Ser Glu Leu Pro Gln
               500                 505                 510
   Gly Thr Thr Val Val Arg Gly Pro Gly Phe Thr Gly Gly Asp Ile Leu
           515                 520                 525
   Arg Arg Thr Asn Thr Gly Gly Phe Gly Pro Ile Arg Val Thr Val Asn
       530                 535                 540
   Gly Pro Leu Thr Gln Arg Tyr Arg Ile Gly Phe Arg Tyr Ala Ser Thr
   545                 550                 555                 560
   Val Asp Phe Asp Phe Phe Val Ser Arg Gly Gly Thr Thr Val Asn Asn
                   565                 570                 575
   Phe Arg Phe Leu Arg Thr Met Asn Ser Gly Asp Glu Leu Lys Tyr Gly
               580                 585                 590
   Asn Phe Val Arg Arg Ala Phe Thr Thr Pro Phe Thr Phe Thr Gln Ile
           595                 600                 605
   Gln Asp Ile Ile Arg Thr Ser Ile Gln Gly Leu Ser Gly Asn Gly Glu
       610                 615                 620
   Val Tyr Ile Asp Lys Ile Glu Ile Ile Pro Val Thr Ala Thr Phe Glu
   625                 630                 635                 640
   Ala Glu Tyr Asp Leu Glu Arg Ala Gln Glu Ala Val Asn Ala Leu Phe
                   645                 650                 655
   Thr Asn Thr Asn Pro Arg Arg Leu Lys Thr Asp Val Thr Asp Tyr His
               660                 665                 670
   Ile Asp Gln Val Ser Asn Leu Val Asp Cys Leu Ser Asp Glu Phe Cys
           675                 680                 685
   Leu Asp Glu Lys Arg Glu Leu Ser Glu Lys Val Lys His Ala Lys Arg
       690                 695                 700
   Leu Ser Asp Glu Arg Asn Leu Leu Gln Asp Pro Asn Phe Arg Gly Ile
   705                 710                 715                 720
   Asn Arg Gln Pro Asp Arg Gly Trp Arg Gly Ser Thr Asp Ile Thr Ile
                   725                 730                 735
   Gln Gly Gly Asp Asp Val Phe Lys Glu Asn Tyr Val Thr Leu Pro Gly
   740 745 750
   Thr Val Asp Glu Cys Tyr Pro Thr Tyr Leu Tyr Gln Lys Ile Asp Glu
   755 760 765
   Ser Lys Leu Lys Ala Tyr Thr Arg Tyr Glu Leu Arg Gly Tyr Ile Glu
   770 775 780
   Asp Ser Gln Asp Leu Glu Ile Tyr Leu Ile Arg Tyr Asn Ala Lys His
   785 790 795 800
   Glu Ile Val Asn Val Pro Gly Thr Gly Ser Leu Trp Pro Leu Ser Ala
   805 810 815
   Gln Ser Pro Ile Gly Lys Cys Gly Glu Pro Asn Arg Cys Ala Pro His
   820 825 830
   Leu Glu Trp Asn Pro Asp Leu Asp Cys Ser Cys Arg Asp Gly Glu Lys
   835 840 845
   Cys Ala His His Ser His His Phe Thr Leu Asp Ile Asp Val Gly Cys
   850 855 860
   Thr Asp Leu Asn Glu Asp Leu Gly Val Trp Val Ile Phe Lys Ile Lys
   865 870 875 880
   Thr Gln Asp Gly His Ala Arg Leu Gly Asn Leu Glu Phe Leu Glu Glu
   885 890 895
   Lys Pro Leu Leu Gly Glu Ala Leu Ala Arg Val Lys Arg Ala Glu Lys
   900 905 910
   Lys Trp Arg Asp Lys Arg Glu Lys Leu Gln Leu Glu Thr Asn Ile Val
   915 920 925
   Tyr Lys Glu Ala Lys Glu Ser Val Asp Ala Leu Phe Val Asn Ser Gln
   930 935 940
   Tyr Asp Arg Leu Gln Val Asp Thr Asn Ile Ala Met Ile His Ala Ala
   945 950 955 960
   Asp Lys Arg Val His Arg Ile Arg Glu Ala Tyr Leu Pro Glu Leu Ser
   965 970 975
   Val Ile Pro Gly Val Asn Ala Ala Ile Phe Glu Glu Leu Glu Gly Arg
   980 985 990
   Ile Phe Thr Ala Tyr Ser Leu Tyr Asp Ala Arg Asn Val Ile Lys Asn
   995 1000 1005
   Gly Asp Phe Asn Asn Gly Leu Leu Cys Trp Asn Val Lys Gly His Val
   1010 1015 1020
   Asp Val Glu Glu Gln Asn Asn His Arg Ser Val Leu Val Ile Pro Glu
   1025 1030 1035 1040
   Trp Glu Ala Glu Val Ser Gln Glu Val Arg Val Cys Pro Gly Arg Gly
   1045 1050 1055
   Tyr Ile Leu Arg Val Thr Ala Tyr Lys Glu Gly Tyr Gly Glu Gly Cys
   1060 1065 1070
   Val Thr Ile His Glu Ile Glu Asp Asn Thr Asp Glu Leu Lys Phe Ser
   1075 1080 1085
   Asn Cys Val Glu Glu Glu Val Tyr Pro Asn Asn Thr Val Thr Cys Asn
   1090 1095 1100
   Asn Tyr Thr Gly Thr Gln Glu Glu Tyr Glu Gly Thr Tyr Thr Ser Arg
   1105 1110 1115 1120
   Asn Gln Gly Tyr Asp Glu Ala Tyr Gly Asn Asn Pro Ser Val Pro Ala
   1125 1130 1135
   Asp Tyr Ala Ser Val Tyr Glu Glu Lys Ser Tyr Thr Asp Gly Arg Arg
   1140 1145 1150
   Glu Asn Pro Cys Glu Ser Asn Arg Gly Tyr Gly Asp Tyr Thr Pro Leu
   1155 1160 1165
   Pro Ala Gly Tyr Val Thr Lys Asp Leu Glu Tyr Phe Pro Glu Thr Asp
   1170 1175 1180
   Lys Val Trp Ile Glu Ile Gly Glu Thr Glu Gly Thr Phe Ile Val Asp
   1185 1190 1195 1200
   Ser Val Glu Leu Leu Leu Met Glu Glu
   1205
<210> 7
   <211> 2160
   <212> DNA
<213> Artificial Sequence
<220>
   <223> Description of Artificial Sequence: modified cry1Ia gene
<400> 7
   atgaaactaa agaatcaaga taagcatcaa agtttttcta gcaatgcgaa agtagataaa 60
   atctctacgg attcactaaa aaatgaaaca gatatagaat tacaaaacat taatcatgaa 120
   gattgtttga aaatgtctga gtatgaaaat gtagagccgt ttgttagtgc atcaacaatt 180
   caaacaggta ttggtattgc gggtaaaata cttggtaccc taggcgttcc ttttgcagga 240
   caagtagcta gtctttatag ttttatctta ggtgagctat ggcctaaggg gaaaaatcaa 300
   tgggaaatct ttatggaaca tgtagaagag attattaatc aaaaaatatc aacttatgca 360
   agaaataaag cacttacaga cttgaaagga ttaggagatg ccttagctgt ctaccatgat 420
   tcgcttgaaa gttgggttgg aaatcgtaat aacacaaggg ctaggagtgt tgtcaagagc 480
   caatatatcg cattagaatt gatgttcgtt cagaaactac cttcttttgc agtgtctgga 540
   gaggaggtac cattattacc gatatatgcc caagctgcaa atttacattt gttgctatta 600
   agagatgcat ctatttttgg aaaagagtgg ggattatcat cttcagaaat ttcaacattt 660
   tataaccgtc aagtcgaacg agcaggagat tattcctacc attgtgtgaa atggtatagc 720
   acaggtctaa ataacttgag gggtacaaat gccgaaagtt gggtacgata taatcaattc 780
   cgtagagaca tgactttaat ggtactagat ttagtggcac tatttccaag ctatgataca 840
   caaatgtatc caattaaaac tacagcccaa cttacaagag aagtatatac agacgcaatt 900
   gggacagtac atccgcatcc aagttttaca agtacgactt ggtataataa taatgcacct 960
   tcgttctctg ccatagaggc tgctgttgtt cgaaacccgc atctactcga ttttctagaa 1020
   caagttacaa tttacagctt attaagtcga tggagtaaca ctcagtatat gaatatgtgg 1080
   ggaggacata aactagaatt ccgaacaata ggaggaacgt taaatatctc aacacaagga 1140
   tctactaata cttctattaa tcctgtaaca ttaccgttca cttctcgaga cgtctatagg 1200
   actgaatcat tggcagggct gaatctattt ttaactcaac ctgttaatgg agtacctagg 1260
   gttgattttc attggaaatt cgtcacacat ccgatcgcat ctgataattt ctattatcca 1320
   gggtatgctg gaattgggac gcaattacag gattcagaaa atgaattacc acctgaagca 1380
   acaggacagc caaattatga atcttatagt catagattat ctcatatagg actcatttca 1440
   gcatcacatg tgaaagcatt ggtatattct tggacgcatc gtagtgcgga ccgtacaaat 1500
   acaattgagc caaatagcat tacacaaata ccattagtaa aagctttcaa tctgtcttca 1560
   ggtgccgctg tagtgagagg accaggattt acaggtgggg atatccttcg aagaacgaat 1620
   actggtacat ttggggatat acgagtaaat attaatccac catttgcaca aagatatcgc 1680
   gtgaggattc gctatgcttc taccacagat ttacaattcc atacgtcaat taacggtaaa 1740
   gctattaatc aaggtaattt ttcagcaact atgaatagag gagaggactt agactataaa 1800
   acctttagaa ctgtaggctt taccactcca tttagctttt tagatgtaca aagtacattc 1860
   acaataggtg cttggaactt ctcttcaggt aacgaagttt atatagatag aattgaattt 1920
   gttccggtag aagtaacata tgaggcagaa tatgattttg aaaaagcgca agagaaggtt 1980
   actgcactgt ttacatctac gaatccaaga ggattaaaaa cagatgtaaa ggattatcat 2040
   attgaccagg tatcaaattt agtagagtct ctatcagatg aattctatct tgatgaaaag 2100
   agagaattat tcgagatagt taaatacgcg aagcaactcc atattgagcg taacatgtag 2160
<210> 8
   <211> 719
   <212> PRT
   <213> Artificial Sequence
<220>
   <223> Description of Artificial Sequence: protein encoded by the modified cry1Ia gene
<400> 8
   Met Lys Leu Lys Asn Gln Asp Lys His Gln Ser Phe Ser Ser Asn Ala
   1 5 10 15
   Lys Val Asp Lys Ile Ser Thr Asp Ser Leu Lys Asn Glu Thr Asp Ile
   20 25 30
   Glu Leu Gln Asn Ile Asn His Glu Asp Cys Leu Lys Met Ser Glu Tyr
   35 40 45
   Glu Asn Val Glu Pro Phe Val Ser Ala Ser Thr Ile Gln Thr Gly Ile
   50 55 60
   Gly Ile Ala Gly Lys Ile Leu Gly Thr Leu Gly Val Pro Phe Ala Gly
   65 70 75 80
   Gln Val Ala Ser Leu Tyr Ser Phe Ile Leu Gly Glu Leu Trp Pro Lys
   85 90 95
   Gly Lys Asn Gln Trp Glu Ile Phe Met Glu His Val Glu Glu Ile Ile
   100 105 110
   Asn Gln Lys Ile Ser Thr Tyr Ala Arg Asn Lys Ala Leu Thr Asp Leu
   115 120 125
   Lys Gly Leu Gly Asp Ala Leu Ala Val Tyr His Asp Ser Leu Glu Ser
   130 135 140
   Trp Val Gly Asn Arg Asn Asn Thr Arg Ala Arg Ser Val Val Lys Ser
   145 150 155 160
   Gln Tyr Ile Ala Leu Glu Leu Met Phe Val Gln Lys Leu Pro Ser Phe
   165 170 175
   Ala Val Ser Gly Glu Glu Val Pro Leu Leu Pro Ile Tyr Ala Gln Ala
   180 185 190
   Ala Asn Leu His Leu Leu Leu Leu Arg Asp Ala Ser Ile Phe Gly Lys
   195 200 205
   Glu Trp Gly Leu Ser Ser Ser Glu Ile Ser Thr Phe Tyr Asn Arg Gln
   210 215 220
   Val Glu Arg Ala Gly Asp Tyr Ser Tyr His Cys Val Lys Trp Tyr Ser
   225 230 235 240
   Thr Gly Leu Asn Asn Leu Arg Gly Thr Asn Ala Glu Ser Trp Val Arg
   245 250 255
   Tyr Asn Gln Phe Arg Arg Asp Met Thr Leu Met Val Leu Asp Leu Val
   260 265 270
   Ala Leu Phe Pro Ser Tyr Asp Thr Gln Met Tyr Pro Ile Lys Thr Thr
   275 280 285
   Ala Gln Leu Thr Arg Glu Val Tyr Thr Asp Ala Ile Gly Thr Val His
   290 295 300
   Pro His Pro Ser Phe Thr Ser Thr Thr Trp Tyr Asn Asn Asn Ala Pro
   305 310 315 320
   Ser Phe Ser Ala Ile Glu Ala Ala Val Val Arg Asn Pro His Leu Leu
   325 330 335
   Asp Phe Leu Glu Gln Val Thr Ile Tyr Ser Leu Leu Ser Arg Trp Ser
   340 345 350
   Asn Thr Gln Tyr Met Asn Met Trp Gly Gly His Lys Leu Glu Phe Arg
   355 360 365
   Thr Ile Gly Gly Thr Leu Asn Ile Ser Thr Gln Gly Ser Thr Asn Thr
   370 375 380
   Ser Ile Asn Pro Val Thr Leu Pro Phe Thr Ser Arg Asp Val Tyr Arg
   385 390 395 400
   Thr Glu Ser Leu Ala Gly Leu Asn Leu Phe Leu Thr Gln Pro Val Asn
   405 410 415
   Gly Val Pro Arg Val Asp Phe His Trp Lys Phe Val Thr His Pro Ile
   420 425 430
   Ala Ser Asp Asn Phe Tyr Tyr Pro Gly Tyr Ala Gly Ile Gly Thr Gln
   435 440 445
   Leu Gln Asp Ser Glu Asn Glu Leu Pro Pro Glu Ala Thr Gly Gln Pro
   450 455 460
   Asn Tyr Glu Ser Tyr Ser His Arg Leu Ser His Ile Gly Leu Ile Ser
   465 470 475 480
   Ala Ser His Val Lys Ala Leu Val Tyr Ser Trp Thr His Arg Ser Ala
   485 490 495
   Asp Arg Thr Asn Thr Ile Glu Pro Asn Ser Ile Thr Gln Ile Pro Leu
   500 505 510
   Val Lys Ala Phe Asn Leu Ser Ser Gly Ala Ala Val Val Arg Gly Pro
   515 520 525
   Gly Phe Thr Gly Gly Asp Ile Leu Arg Arg Thr Asn Thr Gly Thr Phe
   530 535 540
   Gly Asp Ile Arg Val Asn Ile Asn Pro Pro Phe Ala Gln Arg Tyr Arg
   545 550 555 560
   Val Arg Ile Arg Tyr Ala Ser Thr Thr Asp Leu Gln Phe His Thr Ser
   565 570 575
   Ile Asn Gly Lys Ala Ile Asn Gln Gly Asn Phe Ser Ala Thr Met Asn
   580 585 590
   Arg Gly Glu Asp Leu Asp Tyr Lys Thr Phe Arg Thr Val Gly Phe Thr
   595 600 605
   Thr Pro Phe Ser Phe Leu Asp Val Gln Ser Thr Phe Thr Ile Gly Ala
   610 615 620
   Trp Asn Phe Ser Ser Gly Asn Glu Val Tyr Ile Asp Arg Ile Glu Phe
   625 630 635 640
   Val Pro Val Glu Val Thr Tyr Glu Ala Glu Tyr Asp Phe Glu Lys Ala
   645 650 655
   Gln Glu Lys Val Thr Ala Leu Phe Thr Ser Thr Asn Pro Arg Gly Leu
   660 665 670
   Lys Thr Asp Val Lys Asp Tyr His Ile Asp Gln Val Ser Asn Leu Val
   675 680 685
   Glu Ser Leu Ser Asp Glu Phe Tyr Leu Asp Glu Lys Arg Glu Leu Phe
   690 695 700
   Glu Ile Val Lys Tyr Ala Lys Gln Leu His Ile Glu Arg Asn Met
   705 710 715
<210> 9
   <211> 37
   <212> DNA
   <213> Artificial Sequence
<220>
   <223> Description of Artificial Sequence: oligomer used for mutagenesis of the cry1Ba gene
<400> 9
   ggacgcatcg tagtgcggac cgtacgaata cgattgg                          37
<210> 10
   <211> 36
   <212> DNA
   <213> Artificial Sequence
<220>
   <223> Description of Artificial Sequence: oligomer used for mutagenesis of the cry1Ia gene
<400> 10
   ggacgcatcg tagtgcggac cgtacaaata caattg                           36

## Description

### 1.0. Field of the invention

The present invention relates to hybrid toxin fragments, and toxins comprising them, derived from *Bacillus thuringiensis* insecticidal crystal proteins.

### 1.1. Description of the related art

*Bacillus thuringiensis* (hereinafter B.t.) is a gram-positive bacterium that produces insecticidal crystal proteins during sporulation. The crystal (Cry) proteins form a large (over 160 and growing) family of homologous proteins with unique specificities. Each protein is active against only one or a few insect species. Most reported proteins are active against lepidopterans, with a smaller number showing activity against diptera or coleoptera as reviewed in Schnepf *et al.,* 1998). While initially the Cry proteins were classified according to their activity against one of the abovementioned insect orders (Höfte and Whiteley, 1989), the more recent, now commonly accepted classification is based on amino acid homology (Crickmore *et al.,* 1998).

The mode of actions of crystal proteins has been partially elucidated. After ingestion by the insect larvae, crystals dissolve in the midgut environment, releasing the proteins as protoxins of 70-140 kDa. The solubilized protoxins are subsequently processed ("activated") by midgut proteases, resulting in a protease-resistant fragment of about 65 kDa, which is the active toxin. The toxin binds to receptors on epithelial cells of the insect midgut and penetrates the membrane. This eventually leads to lysis of the cells and death of the larvae.

The activity range of a particular delta-endotoxin is to a large extent determined by the occurrence of receptors on the midgut cells of the insect, although solubilization efficiency and proteolytic activition are also factors involved. The importance of binding to receptors is further examplified by the decrease in binding occuring in many instances of resistance to Cry proteins (Ferré *et al.,* 1995).

Structure determination by X-ray cristallography has shown that three different Cry proteins, and probably all Cry proteins, share a common three domain-structure (Li *et al.,* 1991; Grochulski *et al.,* 1995; Morse *et al.,* 1998). If projected on Cry1 sequences, domain I runs from about amino acid residue 28 to 260, domain II from about 260 to 460 and domain III from about 460 to 620. Since the various toxins have different lengths, the borders of the domains can be defined only approximately. A person skilled in this art will be able to find the borders for the various toxins by comparing the amino acid sequences. The N-terminal domain I consists of 7 α-helices and is considered to be inserting (partially) into the target membrane, and forming part of the pore that eventually kills the insect gut epithelial cells. Both domain II and the C-terminal domain III are very variable, and have been shown to determine activity against specific insects. Although it is not yet clear how these domains individually or acting together may determine specificity, there is strong evidence that both can be involved in binding to (putative) receptors (Lee *et al.,* 1995; Dean *et al.,* 1996; de Maagd *et al*., 1 996b; de Maagd *et al.,* 1999).

Exchange of domain III between toxins by *in vivo* recombination of their encoding genes may not only alter specificity of a toxin, but can also result in a hybrid toxin with superior toxicity for certain insects (Bosch *et al.,* 1994; de Maagd *et al.,* 1996a). Intl. Pat. Appl. Publ. No. WO 95/06730 discloses the construction of a hybrid delta-endotoxin consisting of domains I and II of Cry1E, and domain III and protoxin-specific fragment of Cry1C. In bioassays, this hybrid as purified after production in *E*. *coli* is active against *Manduca sexta, Spodoptera exigua,* and *Mamestra brassicae.* When expressed in and purified from a recombinant *Bacillus thuringiensis* strain, the 1E/1 C-hybrid was 1.5 times as active as the most active natural toxin against *S*. *exigua*, Cry1C. The abovementioned patent application also describes a hybrid delta-endotoxin consisting of domains I and II of Cry1Ab, and domain III of Cry1C. When purified from a recombinant *Bacillus thuringiensis* strain, this Cry1 Ab/Cry1 C-hybrid was approximately 6.6 times as toxic as Cry1 C (de Maagd *et al.,* 1996a). Intl. Pat. Appl. Publ. No. WO 98/22595 discloses a number of hybrid toxins consisting of different combinations of fragments of Cry1Ab, Cry1 Ac, Cry1C or Cry1F, of which some have improved acitivity against important pest larvae and/or an extended activity spectrum.

Producing succesfull hybrids is not easy. From the prior art it appears that only a very limited number of hybrids have an improved activity or a broader host-range specificity. In addition there are many possibilities for recombinantly-engineered crystal proteins, in view of the big number of different crystal proteins (more than 160). Further, the effect of the hybrids produced is not predictable.

### 2.0 Summary of the invention

Proteins of the Cry3 (Herrnstadt *et al*., 1986; McPherson *et al*., 1988), Cry7 (Lambert *et al.,* 1992) and Cry8 (Sato *et al*., 1994) classes were found to be active against insects of the order Coleoptera (beetles). Cry3A is the singlemost active protein for the important potato pest Colorado potato beetle (CPB), *Leptinotarsa decemlineata* (Say).

It is known that resistance to the endotoxins may occur. Accordingly , resistance to Cry3A may occur in CPB in response to its exposure to Cry3A in transgenic potato. Actually, resistant CPB has already been disclosed. Therefore, it is desirable to have an alternative or replacement for Cry3A.

The present inventors have found specific hybrid crystal proteins which have a high activity against CPB and therefore can be used as a replacement for Cry3A. The hybrids of the invention comprise domains derived from Cry1Ia and Cry1Ba.

Cry1 proteins are generally active against lepidopterans (larvae of moths and butterflies). Cry1 B and Cry1 I have been shown to also have some activity against coleopterans, among which CPB, although their toxicity for CPB is much lower than that of Cry3A (Tailor *et al*., 1992; Bradley *et al*., 1995).

The toxicity of the hybrid crystal proteins of the invention against CPB, as compared to the parental proteins Cry1Ba and Cry1Ia, is surprisingly higher. Additionally a number of the hybrids of the invention have retained the high activity against some Lepidopterans, making them toxins with meaningful dual activity for Coleopterans and Lepidopterans.

According to the present invention there is provided a B.t. hybrid toxin fragment comprising structural domains I, II and III in this order starting from the N-terminal, wherein the domains are derived from at least two different Cry proteins, domain I is domain I of any of *B. t.* Cry protein or a part of said domain or a peptide substantially similar to said domain , domain II is domain II of Cry1Ia or a part of said domain or a peptide substantially similar to said domain, and domain III is domain III of Cry1Ba or a part of said domain or a peptide substantially similar to said domain. Preferred is a fragment which comprises domain I of Cry1Ia or Cry1Ba or a part of said domain or a peptide substantially similar to said domain, domain II of Cry1Ia or a part of said domain or a peptide substantially similar to said domain, and domain III of Cry1Ba or a part of said domain or a peptide substantially similar to said domain.

The term " or a peptide substantially similar to said domain" should be understood to mean a peptide having an amino acid sequence which is at least 85% similar to the sequence of the domain. It is preferred that the degree of similarity is at least 90%.

In the context of the present invention, two amino acid sequences with at least 85% or 90% similarity to each other have at least 85% or 90% identical or conservatively replaced amino acid residues in a like position when aligned optimally allowing for up to 6 gaps with the *proviso* that in respect of the gaps a total not more than 15 amino acid residues are affected. For the purpose of the present invention conservative replacements may be made between amino acids with the following groups:
1. Serine and Threonine;
2. Glutamic acid and Aspartic acid;
3. Arginine and Lysine
4. Asparagine and Glutamine
5. Isoleucine, Leucine, Valine, and Methionine;
6. Phenylalanine, Tyrosine, and Tryptophan
7. Alanine and Glycine
By "or a part of said domain" is meant a peptide comprised by the said domain and having at least 80% of the consecutive sequence thereof.

It is most particularly preferred that the toxin fragment according to the invention comprises either:
1. An amino acid sequence from about amino acid 20 to about amino acid 641 in SEQ ID NO:2 or
2. An amino acid sequence from about amino acid 20 to about amino acid 632 in SEQ ID NO:4.

The invention also includes a hybrid toxin comprising the above disclosed fragment or a toxin at least 85% similar to such a hybrid toxin which has substantially similar insecticidal activity.

The hybrid toxin comprises the three structural domains as defined above and generally may comprise a pro-toxin segment at the carboxyl end, which is not toxic and is thought to be important for crystal formation. SEQ ID NO:2 and 4 comprise such pro-toxin segment. Further, the hybrid toxin may comprise a lead sequence, being amino acids 1-19 in SEQ ID NO:2 and 4.

The invention still further includes pure proteins which are at least 90% identical to the toxin fragments or hybrid toxins according to the invention.

The invention still further includes recombinant DNA comprising a sequence encoding a protein having an amino acid sequence of the above disclosed toxins or fragments thereof.

In a preferred embodiment the invention provides recombinant DNA comprising the sequence as shown in SEQ ID NO:1 or 3 or DNA similar thereto encoding a substantially similar protein.

In a more preferred embodiment the invention provides recombinant DNA comprising the sequence from about nucleotide 170 to about 1929 in SEQ ID NO:1 or from about nucleotide 147 to about 1896 in SEQ ID NO:3.

By similar DNA is meant a test sequence which is capable of hybridizing to the inventive recombinant sequence. When the test and inventive sequences are double stranded the nucleic acid constituting the test sequence preferably has a Tₘ within 20°C of that of the inventive sequence. In the case that the test and inventive sequences are mixed together and denantured simultaneously, The Tₘ values of the sequences are preferably within 10°C of each other. More preferably the hybridization is performed under stringent conditions, with either the test or inventive DNA preferaby being supported. Thus either a denatured test or inventive sequence is preferably first bound to a support and hybridization is effected for a specified period of time at a temperature of between 50 and 70 °C in double strength citrate buffered saline containing 0.1 % SDS followed by rinsing of the support at the same temperature but with a buffer having a reduced SSC concentration. Depending upon the degree of stringency required, and thus the degree of similarity of the sequences, such reduced concentration buffers are typically single strength SSC containing 0.1 % SDS. Sequences having the greatest degree of similarity are those the hybridization of which is least affected by washing in buffers of reduced concentration. It is most preferred that the test and inventive sequences are so similar that the hybridization between them is substantially unaffected by washing or incubation in one tenth strength sodium citrate buffer containing 0.1 % SDS.

The recombinant DNA may further encode a protein having herbicide resistance, plant growth-promoting, anti-fungal , anti-bacterial, anti-viral and/or anti-nematode properties. In the case that the DNA is to be introduced into a heterologous organism it may be modified to remove known mRNA instability motifs (such as AT rich regions) and polyadenylation signals, and/or codons which are preferred by the organism into which the recombinant DNA is to be inserted may be used so that expression of the thus modified DNA in the said organism yields substantially similar protein to that obtained by expression of the unmodified recombinant DNA in the organism in which the protein components of the hybrid toxin or toxin fragments are endogenous.

The invention still further includes a DNA sequence which is complementary to one which hybridizes under stringent conditions with the recombinant DNA according to the invention.

Also included in the present invention are: a vector containing such a recombinant (or complementary thereto) DNA sequence; a plant or micro-organism which includes, and enables expression of such DNA; plants transformed with such DNA; the progeny of such plants which contain the DNA stably incorporated and hereditable in a Mendelian manner, an/or the seeds of such plants and such progeny.

The invention still further includes protein derived from expression of the said DNA, and insecticidal protein produced by expression of the recombinant DNA within plants transformed therewith.

The invention still further includes an insecticidal composition containing one or more of the toxin fragments or toxins comprising them according to the invention; a process for combatting insects which comprises exposing them to such fragments or toxins or compositions, and an extraction process for obtaining insecticidal proteins from organic material containing them comprising submitting the material to maceration and solvent extraction.

The invention will be further apparent from the following description, which describes the production of Bt hybrid toxin fragments according to the invention, taken in conjunction with the associated drawings and sequence listings.

A person skilled in the art may use different methods to construct the hybrid toxin genes of the invention. Domain encoding regions may be exchanged between two homologous, though different genes by exchanging fragments through restriction enzyme digestion and subsequent ligation, if the same restriction enzyme recognition sites are present at the same position in the two genes. If suitable restriction enzyme recognition sites are not available, new sites can be created at homologous positions in the two genes by various methods of site-directed mutagenesis, as described in the examples below for construction of a common Rsrll-site using DNA oligomers SEQ ID: 9 and SEQ ID: 10.

Alternatively, fragments that are to be combined in a hybrid toxin gene may be produced by PCR-mediated amplification using the original genes as templates, taking care that the ends of the fragments contain compatible restriction enzyme digestion sites. Furthermore, a hybrid toxin encoding DNA fragment may be produced *by in vitro* recombination between overlapping, partially homologous DNA fragments as performed in so-called DNA shuffling experiment (Crameri et al, 1 998; Zhao et al., 1998). Also, hybrid toxin encoding DNA fragments may be produced by *in vivo* recombination between two homologous DNA fragments that have been cloned in tandem on a single plasmid (Bosch et al., 1994), followed by screening for the desired recombination events.

### 3.0 Brief description of the drawings

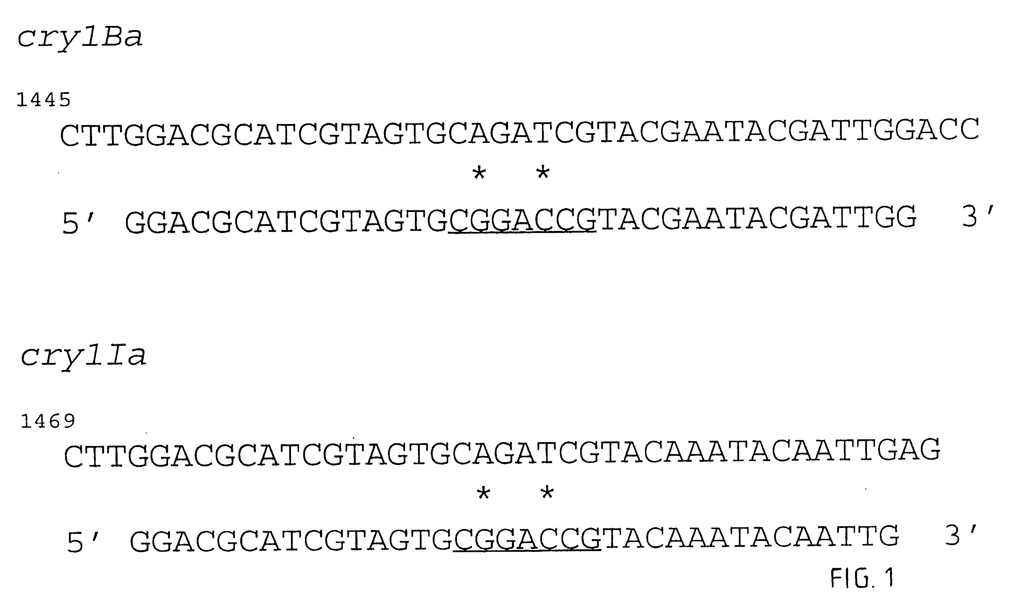

**Figure 1** shows the sequence of the relevant part of the *cry1Ba* (top) and *cry1Ia* (bottom) genes, respectively, aligned with the respective oligomers (SEQ ID NO:9 and 10) used for mutagenesis in order to produce a common *Rsr*ll restriction enzyme recognition site in both genes. Mutated nucleotides are indicated by asterisks and the newly produced *Rsr*II recognition sites are underlined.

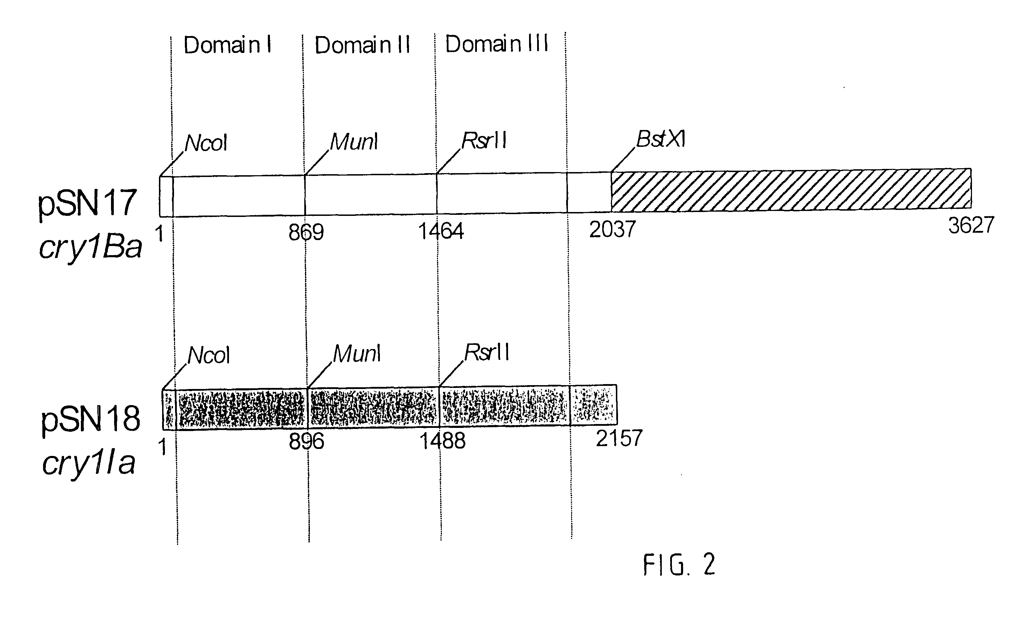

**Figure 2** shows both the *cry1Ia* gene (solid bar) as well as the *cry1Ba* fragment (open bar) presented as they occur in expression vectors pSN18 and pSN17, respectively. Both genes are inserted in the pKK233-2 derived expression vector for *E. coli,* pBD12. For construction of pSN18 the 3' part of the *cry1Ba* gene, encoding the C-terminal part of the protoxin-specific fragment, was replaced by the corresponding part of the *cry1Ca* gene (base pairs 2038-3627; dashed bar). The positions of the common *Nco*l and *Mun*I restriction sites, as well as of the *Rsr*II sites derived by mutagenesis, and the *BstX*I*-*site used for exchange of the 3'end of the *cry1Ba* gene are indicated, with nucleotide position numbers below the respective genes.

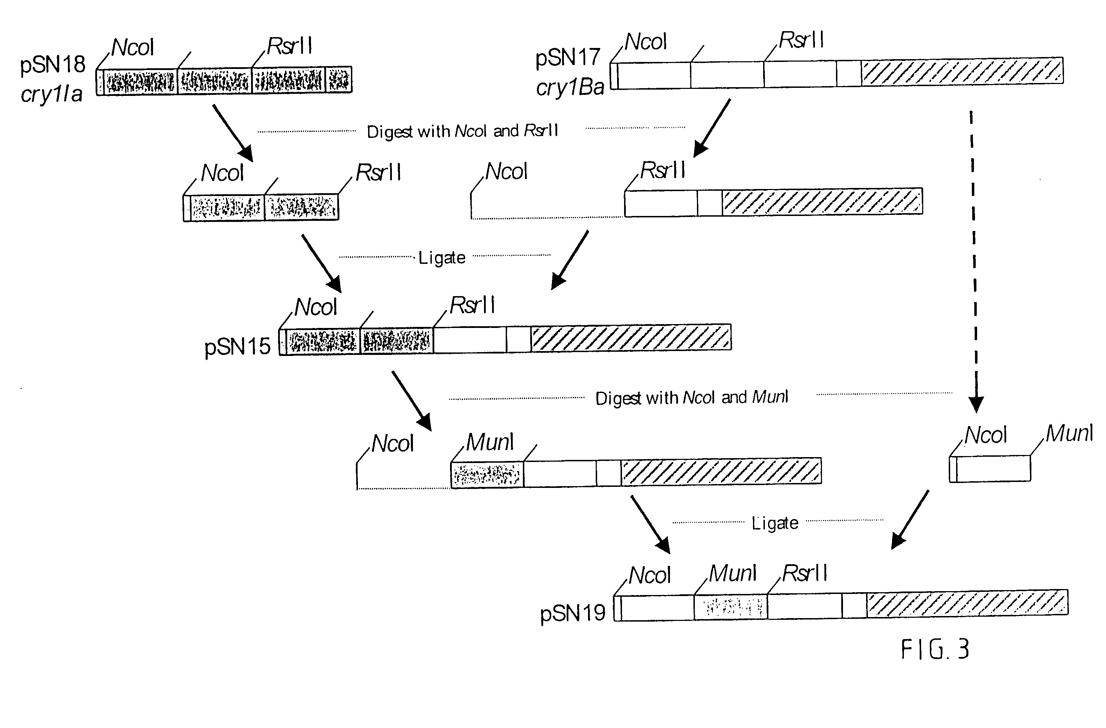

Figure 3 shows in a scheme the construction of hybrid gene SN1 5 from *cry1Ba* (pSN17) and *cry1Ia* (pSN18), and the subsequent construction of hybrid gene SN19 from *cry1Ba* (pSN17) and pSN15.

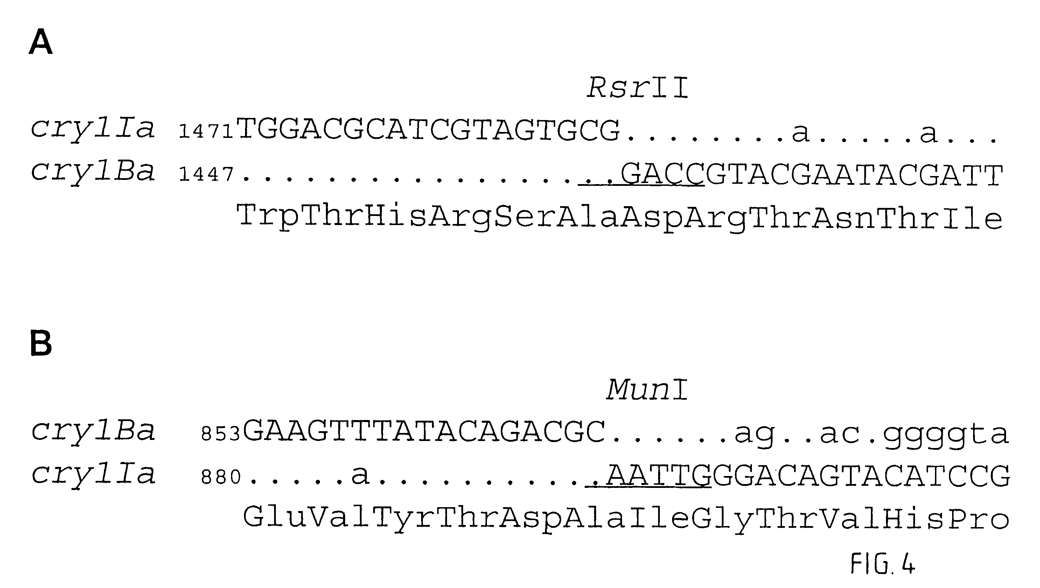

Figure 4 shows the alignment of *cry1Ba* and *cry1Ia* nucleotide sequences, as present in plasmids pSN17 and pSN18, respectively, around the junctions between the domain II and III (A) and between domain I and II (B). Nucleotide sequences as present in the hybrid genes of SN15 and pSN19 (A) or of pSN19 alone (B) are given in capitals, while identical nucleotides in the DNA that is not present in SN15 and SN19 is represented by dots. The position of the common *Rsr*ll and *Mun*l recognition sites are underlined. The site of crossover in the hybrids is represented by the shift of the fully written out nucleotide sequence from top strand to bottom strand in this recognition sites. The amino acid sequence of the resulting hybrid proteins is given in three-letter code below each alignment.

### 4.0 Brief description of the sequence identifiers

**SEQ ID NO:1** shows the nucleotide sequence of the hybrid protoxin gene SN15.
**SEQ ID NO:2** shows the amino acid sequence of the protein encoded by the gene SN 15 shown in SEQ ID NO:1.
**SEQ ID NO:3** shows the nucleotide sequence of the hybrid protoxin gene SN19.
**SEQ ID NO:4** shows the amino acid sequence of the protein encoded by the gene SN 19 shown in SEQ ID NO:3.
**SEQ ID NO:5** shows the nucleotide sequence of the modified *cry1Ba* gene as used in expression vector pSN 17.
**SEQ ID NO:6** shows the amino acid sequence of the protein encoded by the *cry1Ba* gene shown in SEQ ID NO:5.
**SEQ ID NO:7** shows the nucleotide sequence of the modified *cry1Ia* gene as used in expression vector pSN 18.
**SEQ ID NO:8** shows the amino acid sequence of the protein encoded by the *cry1Ia* gene shown in SEQ ID NO:7.
**SEQ ID NO:9** shows the nucleotide sequence of the oligomer used for mutagenesis of the *cry1Ba* gene.
**SEQ ID NO:10** shows the nucleotide sequence of the oligomer used for mutagenesis of the *cry1Ia* gene.

### 5.0 Examples

### 5.1 DNA manipulations

All recombinant DNA techniques are as described by Ausubel et al. (1997). Mutagenesis, restriction enzyme digestion and ligation are performed according the instructions of the manufacturers. DNA sequencing is performed by the dideoxytriphosphate methode with fluorescent dyes attached to the dideoxynucleotides. Analysis is automated by using an Applied Biosystems 370A nucleotide sequence analyzer.

### 5.2 Expression vectors

All Cry protein expression vectors are based on pBD12, a derivative of pKK233-2 (Bosch *et al.,* 1 994). For expression of Cry3Aa protein, the full *cry3Aa* gene is cloned into pBD12, giving expression plasmid pMH10. For cloning purposes both *cry1Ba* as well as *cry1Ia* are mutagenized in order to contain a *Nco*I-site overlapping with the start codon. For production of Cry1Ba protein, a *Nco*I*-BstX*I (bases 1-1977) fragment of *cry1Ca* in pBD150 (Bosch *et al*., 1994) is replaced by the corresponding fragment of *cry1Ba* (bases 1-2037), resulting in *cry1Ba* expression vector pMH19. pMH19 therefor contains the 5'active toxin encoding part of Cry1Ba and 3' protoxin specific part of Cry1Ca. Cry1Ia expression vector pBD172 contains the full *cry1Ia* gene with the Spel-site (base 2180) fused to the Spel-site in the polylinker of pBluescript SK⁺.

### 5.3 Mutagenesis of cry1Ba and cry1la

In order to be able to directly exchange the domain III encoding regions between *cry1Ba* and *cry1Ia*, a new common restriction enzyme recognition site is made in both genes by site directed mutagenesis. Complementary mutagenic oligonucleotide pairs are used to create unique *Rsr*II-sites at positions 1464 and 1488 of *cry1Ba* (pMH19) and *cry1Ia* (pBD172), respectively (see Fig. 1), using the using the QuickChange™ kit (Stratagene). These mutations do not change the encoded proteins. This results in two new expression plasmids, pSN17 (Cry1Ba, Seq. ID NO: 5) and pSN18 (Cry1Ia, Seq. ID NO: 7), respectively (Fig. 2). The unique *Rsr*II restriction sites at the border of the domain II and domain III encoding regions, together with the common *Mun*I-sites at the border between the domain I and domain II encoding regions allowed simple swapping of domain encoding regions between the two genes.

### 5.4 Construction of hybrid toxins

Fig. 3 schematically shows the construction of two novel hybrid toxins. Both pSN18 and pSN17 are digested with *Nco*I and *Rsr*II. Subsequently, the 1488 base fragment of pSN18, containing the domain I and II encoding fragment of *cry1Ia* is ligated into the corresponding sites of the pSN17-derived fragment containing the 3'portion of *cry1Ba.* This results in plasmid pSN 15 encoding a 1Ia/1Ia/1Ba-hybrid (Seq. ID NO:1). The nucleotide sequence of the cross-over region with the encoded amino acid sequence is shown in Fig. 4A. Subsequently a *Nco*l*-Mun*l (base 1-896) fragment encoding domain I of Cry1Ia from pSN15 is replaced by the corresponding fragment encoding domain I of Cry1Ba, derived from pSN17. This results in 1Ba/1la/1Ba-hybrid encoding plasmid pSN19 (Seq. ID. NO:3). The nucleotide sequence of the crossover region with the encoded amino acid sequence is shown in Fig. 4B.

### 5.5 Protein isolation and insect bioassays.

For large-scale production, all parental and hybrid protoxins are expressed in *E. coli* strain XL-1 and extracted as described earlier (Bosch *et al.,* 1994). Solubilized protoxins are dialyzed overnight in 25 mM NaHCO₃, 100 mM NaCl, pH10. Protein concentrations are estimated by SDS-PAGE (sodium dodecylsulphate polyacrylamide gel electrophoresis). To test toxicity to Colorado potato beetle (CPB), leaflets of greenhouse grown potato culitivar Desiree are dipped in toxin dilutions in water containing 0.01 % Tween-20. After drying of the leaves to the air they are transferred to petri dishes and 10 neonate CPB larvae are placed on each leaf. After incubation for two days at 28°C, the leaves are replaced by fresh leaves dipped in identical protoxin dilutions. Mortality is scored after 4 days. LC₅₀ (concentration with 50% mortality) and 95% fiducial limits are determined by Probit analysis of results from three or more independent experiments, using the PoloPC computer program (Russel *et al.,* 1977).

### 5.6 Toxicity of wild type and hybrid proteins to Colorado potato beetle

Table 1 shows the toxicity of the hybrid proteins SN15 and SN19 against Colorado potato beetle (CPB), as compared to the parental proteins Cry1Ba and Cry1Ia, and to the most CPB-active natural toxin available, Cry3A. 1Ia/1Ia/1Ba-hybrid SN15 shows slightly higher toxicity (lower LC50) than its best parental toxin, Crylla, on a per weight basis. When considering that the molecular weight of the SN15 protein is considerably larger than that of Cry1Ia, it follows that SN15 performs even better on a per mol basis. 1 Ba/1la/1Ba-hybrid protein SN19 is even considerably more toxic than SN15.

**Table 1 .**

| Toxicity of wild type and hybrid protoxins to Colorado potato beetles. | | | | |
|---|---|---|---|---|
| Protoxin | LC₅₀^{a} | 95% fiducial limits^{a} | MW^{b} | Relative toxicity^{c} |
| | | | | |
| MH10 (Cry3A) | 1.8 | 1.4-2.5 | 74.0 | 100 |
| SN17 (Cry1Ba) | 142 | 105-198 | 137.4 | 1 |
| SN18 (Cry1Ia) | 34 | 23-47 | 81.3 | 6 |
| SN 15 | 22 | 14-35 | 138.0 | 15 |
| SN19 | 8 | 5-11 | 137.2 | 42 |

| | | | | |
|---|---|---|---|---|
| ^{a}Concentration in microgram per milliliter of dipping solution. LC₅₀: concentration which leads to 50% mortality; | | | | |
| ^{b}Approximate molecular weight in kiloDaltons. | | | | |
| ^{c}Relative toxicity on molar basis in percents, with toxicity of Cry3A set at 100%. | | | | |

Although the present invention has been particularly described for the production of SN 15 and SN 19 hybrid toxins, the skilled scientist will appreciate that other hybrid toxins with improved insecticidal characteristics may be produced according to the invention. Hybrid toxins containing the combination of Cry1Ia domain II and Cry1Ba domain III, with various combinations of domain I and C-terminal extensions may be made. Moreover, the gene encoding SN15, SN19 and/or other hybrids may be transferred into strains of B.t. and/or integrated into the chromosome of strains of B.t., of other bacteria or be introduced into plant genomes to provide for in situ insecticidal activity within the plant per se. In this regard, it is particularly preferred that the recombinant DNA encoding the toxins is modified in that sequences which are detrimental to high level expression in plants are removed and in that codons which are preferred by the plant are used. This should lead to production in the plant of a substantially similar protein to that obtained by expression of the unmodified recombinant DNA in the organism in which the protein components of the hybrid toxins or toxin fragments are endogenous.

### 6.0 References

The following references, have been cited hereinbefore:
Intl. Pat. Appl. Publ. No. WO 95/06730, published 9 March 1995
Intl. Pat. Appl. Publ. No. WO 98/22595, published 28 May 1998
Ausubei, F. *et al.* (1997) *Current Protocols in Molecular Biology.* John Wiley & Sons.
Bosch, D., Schipper, B., van der Kleij, H., de Maagd, R. and Stiekema, W. (1994) Recombinant *Bacillus thuringiensis* crystal proteins with new properties: possibilities for resistance management. *Bio*/*technology,* **12**, 915-918.
Bradley, D., Harkey, M.A., Kim, M.K., Biever, K.D. and Bauer, L.S. (1995) The insectidal CrylB crystal protein of *Bacillus thuringiensis* ssp. *thuringiensis* has dual specificity to Coleopteran and Lepidopteran larvae. *Journal of Invertebrate Pathology,* **65**, 162-173.
Crameri, A., S. A. Raillard, E. Bermudez, and W. P. C. Stemmer. 1998. DNA shuffling of a family of genes from diverse species accelerates directed evolution. Nature. 391 (6664):288-291 .
Crickmore, N., Zeigler, D.R., Feitelson, J., Schnepf, E., VanRie, J., Lereclus, D., Baum, J. and Dean, D.H. (1998) Revision of the nomenclature for the Bacillus thuringiensis pesticidal crystal proteins. *Microbiology and molecular biology reviews,* **62**, 807&.
de Maagd, R.A., Bakker, P.L., Masson, L., Adang, M.J., Sangadala, S., Stiekema, W. and Bosch, D. (1999) Domain III of the *Bacillus thuringiensis* delta-endotoxin Cry1Ac is involved in binding to Manduca sexta brush border membranes and to its purified aminopeptidase N. *Molecular microbiology,* **31**, 463-471.
de Maagd, R.A., Kwa, M.S.G., van der Klei, H., Yamamoto, T., Schipper, B., Vlak, J.M., Stiekema, W.J. and Bosch, D. (1996a) Domain III substitution in *Bacillus thuringiensis* delta-endotoxin CrylA(b) results in superior toxicity for *Spodoptera exigua* and altered membrane protein recognition. *Applied & Environmental Microbiology,* **62**, 1537-1543.
de Maagd, R.A., van der Klei, H., Bakker, P.L., Stiekema, W.J. and Bosch, D. (1996b) Different domains of *Bacillus thuringiensis* delta-endotoxins can bind to insect midgut membrane proteins on ligand blots. *Applied & Environmental Microbiology,* **62**, 2753-2757.
Dean, D.H., Rajamohan, F., Lee, M.K., Wu, S.J., Chen, X.J., Alcantara, E. and Hussain, S.R. (1996) Probing the mechanism of action of *Bacillus thuringiensis* insecticidal proteins by site directed mutagenesis: a minireview. *Gene,* **179**, 111-117.
Ferré, J., Escriche, B., Bel, Y. and van Rie, J. (1995) Biochemistry and genetics of insect resistance to *Bacillus thuringiensis* insecticidal crystal proteins. *FEMS Microbiology Lett.,* **132**, 1-7.
Grochulski, P., Masson, L., Borisova, S., Pusztai-Carey, M., Schwartz, J.L., Brousseau, R. and Cygler, M. (1995) *Bacillus thuringiensis* CrylA(a) insecticidal toxin - crystal structure and channel formation. *Journal of Molecular Biology*, **254**, 447-464.
Herrnstadt, C., Soares, G.G., Wilcox, E.R. and Edwards, D.L. (1986) A new strain of Bacillus thuringiensis with activity against coleopteran insects. *Biotechnology,* **4**, 305-308.
Höfte, H. and Whiteley, H.R. (1989) Insecticidal crystal proteins of *Bacillus thuringiensis. Microbiol. Rev.,* **53**, 242-255.
Lambert, B., Höfte, H., Annys, K., Jansens, S., Soetaert, P. and Peferoen, M. (1992) Novel *Bacillus thuringiensis* insecticidal crystal protein with a silent activity against coleopteran larvae. *Applied And Environmental Microbiology*, **58**, 2536-2542.
Lee, M.K., Young, B.A. and Dean, D.H. (1995) Domain III exchanges of *Bacillus thuringiensis* Cry1A toxins affect binding to different Gyspy Moth midgut receptors. *Biochemical and Biophysical Research Communications,* **216**, 306-312.
Li, J., Carroll, J. and Ellar, D.J. (1991) Crystal structure of insectidicidal delta-endotoxin from *Bacillus thuringiensis* at 2.5 A resolution. *Nature (London),* **353**, 815-821.
McPherson, S., Perlak, F., Fuchs, R., Marrone, P., Lavrik, P. and Fischhoff, D. (1988) Characterization of the coleopteran-specific protein gene of *Bacillus thuringiensis* var. *tenebrionis. Biotechnology,* **6**, 61-66.
Morse, R.J., Powell, G., Ramalingam, V., Yamamoto, T. and Stroud, R.M. (1998) Crystal structure of Cry2Aa from *Bacillus thuringiensis* at 2.2 Angstroms: structural basis of dual specificity. In lizuka, T. (ed.) *Vllth International Colloquium on Invertebrate Pathology and Microbial Control and IVth International Conference on Bacillus thringiensis. ,* Sapporo, pp. 1-2.
Russel, R.M., Robertson, J.L. and Savin, N.E. (1977) POLO: a new computer program for Probit analysis. *ESA Bulletin,* **23**, 209-213.
Sato, R., Takeuchi, K., Ogiwara, K., Minami, M., Kaji, Y., Suzuki, N., Hori, H. and Asano, S. (1994) Cloning, heterologous expression, and localization of a novel crystal protein gene from bacillus-thuringiensis serovar japonensis strain buibui toxic to scarabaeid insects. *Curr Microbiol,* **28**, 15-19.
Schnepf, E., Crickmore, N., van Rie, J., Lereclus, D., Baum, J., Feitelson, J., Zeigler, D.R. and Dean, D.H. (1998) *Bacillus thuringiensis* and its pesticidal crystal proteins. *Microbiology and molecular biology reviews,* **62**, 775-806.
Tailor, R., Tippet, J., Gibb, G., Pells, S., Pike, D., Jordan, L. and Ely, S. (1992) Identification and characterization of a novel *Bacillus thuringiensis* delta-endotoxin entomocidal to coleopteran and lepidopteran larvae. *Mol. Microbiol*., **6**, 1211-1217.
Zhao, H. M., L. Giver, Z. X. Shao, J. A. Affholter, and F. H. Arnold. 1998. Molecular evolution by staggered extension process (StEP) in vitro recombination. Nature biotechnology. **16**(3):258-261.

## Claims

1. *Bacillus thuringiensis* hybrid toxin fragment comprising structural domains I, II and III in this order starting from the N-terminal, domain I running from about amino acid residue 28 to 260, domain 11 running from about amino acid residue 260 to 460 and domain III running from about amino acid residue 460 to 620, if projected on Cry1 sequences, wherein the domains are derived from at least two different Cry proteins, domain I is domain I of Cry1Ia or Cry1Ba or a peptide comprised by said domain and having at least 80% of the consecutive sequence thereof or a peptide having an amino acid sequence which is at least 85 % identical to the sequence of said domain, domain II is domain 11 of Cry1Ia or a peptide comprised by said domain and having at least 80% of the consecutive sequence thereof or a peptide having an amino acid sequence which is at least 85% identical to the sequence of said domain, and domain III is domain III of Cry1Ba or a peptide comprised by said domain and having at least 80% of the consecutive sequence thereof or a peptide having an amino acid sequence which is at least 85% identical to the sequence of said domain.

2. Toxin fragment according to claim 1 comprising an amino acid sequence from about amino acid 20 to about amino acid 641 as shown in SEQ ID NO:2 or an amino acid sequence from about amino acid 20 to about amino acid 632 as shown in SEQ ID NO:4.

3. A hybrid toxin comprising the fragment of any one of the preceding claims ,

4. A toxin according to claim 3 comprising an amino acid sequence as shown in SEQ ID NO:2 or SEQ ID NO:4.

5. Recombinant DNA comprising a sequence encoding a protein having an amino acid sequence of the proteins as claimed in any one of claims 1-4.

6. Recombinant DNA according to claim 5 comprising the sequence as shown in SEQ ID NO: 1 or 3 or DNA similar thereto encoding a substantially similar protein.

7. Recombinant DNA according to claim 5 comprising the nucleotide sequence from about nucleotide 170 to about 1929 in SEQ ID NO:1 or from about 147 to about 1896 in SEQ ID NO:3.

8. Recombinant DNA according to any one of claims 5 to 7 which is modified in that known mRNA instability motifs or polyadenylation signals are removed and/or codons which are preferred by the organism into which the recombinant DNA is to be inserted are used so that expression of the thus modified DNA in the said organism yields substantially similar protein to that obtained by expression of the unmodified recombinant DNA in the organism in which the protein components of the hybrid toxin or toxin fragments are endogenous.

9. A DNA sequence which is complementary to one which hybridizes under stringent conditions with the DNA of any one of claims 5 to 8.

10. A vector containing a DNA sequence as claimed in any one of claims 5 to 9.

11. A plant or micro-organism which includes, and enables expression of, the DNA of any one of claims 5 to 9 or the vector of claim 10.

12. Plants transformed with recombinant DNA as claimed in any one of claims 5 to 9, the progeny of such plants which contain the DNA stably incorporated and hereditable in a Mendelian manner, and/or the seeds of such plants and such progeny.

13. Protein derived from expression of the DNA as claimed in any one of claims 5 to 9 and insecticidal protein produced by expression of the recombinant DNA within plants as claimed in claim 12.

14. An insecticidal composition containing one or more of the proteins as claimed in any one of claims 1 to 4 and 13.

15. A process for combatting insects which comprises exposing them to proteins or compositions as claimed in any one of claims 1 to 4, 13 and 14 or the micro-organism of claim 11.

## Patentansprüche

1. Hybridtoxinfragment von Bacillus thuringiensis umfassend Domänenstrukture I, II und III, in dieser Ordnung von dem N-Terminal anfangend, wobei Domäne I von ungefähr Aminosäurerest 28 bis 260 läuft, Domäne II von ungefähr Aminosäurerest 260 bis 460 läuft und Domäne III von ungefähr Aminosäurerest 460 bis 620 läuft, falls auf Cry1 Sequenzen projektiert, wobei die Domänen von zumindest zwei verschiedenen Cry Proteinen abgeleitet sind, Domäne I ist Domäne I von Cry1Ia oder Cry1Ba oder ein Peptid umfassend ein Teil der genannten Domäne und mit zumindest 80% deren aufeinanderfolgenden Sequenz oder ein Peptid mit einer Aminosäuresequenz die zumindest 85% identisch mit der Sequenz der Domäne ist, Domäne II ist Domäne II von Cry1Ia oder ein Peptid umfassend ein Teil der genannten Domäne und mit zumindest 80% deren aufeinanderfolgenden Sequenz oder ein Peptid mit einer Aminosäuresequenz die zumindest 85% identisch mit der Sequenz der genannten Domäne ist, und Domäne III ist Domäne III von Cry1Ba oder ein Peptid umfassend ein Teil der genannten Domäne und mit zumindest 80% deren aufeinanderfolgenden Sequenz oder ein Peptid mit einer Aminosäuresequenz die zumindest 85% identisch mit der Sequenz der genannten Domäne ist.

2. Toxinfragment nach Anspruch 1, umfassend eine Aminosäuresequenz von ungefähr Aminosäure 20 bis ungefähr Aminosäure 641, wie in SEQ ID NO:2 dargestellt oder eine Aminosäuresequenz von ungefähr Aminosäure 20 bis ungefähr Aminosäure 632, wie in SEQ ID NO:4 gezeigt.

3. Hybridtoxin umfassend das Fragment eines der hervorgehenden Ansprüche.

4. Toxin nach Anspruch 3, umfassend eine Aminosäuresequenz, wie in SEQ ID NO:2 oder SEQ ID NO:4 gezeigt.

5. Rekombinantes DNA umfassend eine für ein Protein codierende Sequenz mit einer Aminosäuresequenz der Proteine nach einem der Ansprüche 1-4.

6. Rekombinantes DNA nach Anspruch 5, umfassend die Sequenz wie in SEQ ID NO:1 oder 3 gezeigt oder gleichartiges DNA codierend für ein hauptsächlich ähnliches Protein.

7. Rekombinantes DNA nach Anspruch 5, umfassend die Nucleotidsequenz von ungefähr Nucleotid 170 bis ungefähr 1929 in SEQ ID NO:1 oder von ungefähr 147 bis ungefähr 1896 in SEQ ID NO:3.

8. Rekombinantes DNA nach einem der Ansprüche 5 bis 7, **dadurch** modifiziert, daß bekannte mRNA Instabilitätsmotive oder Polyadenylationssignale entfernt werden, und/oder Kodone die durch das Organismus worin das rekombinante DNA eingeführt werden muß, bevorzugt werden, benutzt werden, so daß Expression von dem also modifizierten DNA in dem Organismus ein hauptsächlich gleiches Protein ergibt als das durch Expression des unmodifizierten rekombinanten DNA in dem Organismus erhalten, worin die Proteinkomponente des Hybridtoxins oder Toxinfragmente endogen sind.

9. DNA-Sequenz komplementär an der, welche unter stringenten Umständen mit dem DNA nach einem der Ansprüche 5 bis 8 hybridisiert.

10. Vektor enthaltend eine DNA-Sequenz nach einem der Ansprüche 5 bis 9.

11. Pflanze oder Mikroorganismus die das DNA nach einem der Ansprüche 5 bis 9 oder dem Vektor nach Anspruch 10 einschließt und Expression davon ermöglicht.

12. Pflanzen mit rekombinantem DNA nach einem der Ansprüche 5 bis 9 transformiert, die Nachkommenschaft solcher Pflanzen die das DNA stabil inkorporiert und erblich auf eine Mendelion-Weise enthalten, und/oder die Samen solcher Pflanzen und solcher Nachkommenschaft.

13. Protein von der Expression von dem DNA nach einem der Ansprüche 5 bis 9 abgeleitet und Insektizidprotein durch die Expression vom rekombinanten DNA in den Pflanzen nach Anspruch 12 produziert.

14. Insektizidzusammensetzung enthaltend ein oder mehrere der Proteine nach einem der Ansprüche 1 bis 4 und 13.

15. Verfahren zum Bekämpfen von Insekten umfassend das Bloßstellen an Proteinen oder Zusammensetzungen nach einem der Ansprüche 1 bis 4, 13 und 14 oder Mikroorganismus nach Anspruch 11.

## Revendications

1. Fragment de toxine hybride de *Bacillus thuringiensis* comprenant les domaines structurels I, II et III dans cet ordre en partant de la terminaison N, le domaine I s'étendant environ des résidus d'acides aminés 28 à 260, le domaine II s'étendant environ des résidus d'acides aminés 260 à 460 et le domaine III s'étendant environ des résidus d'acides aminés 460 à 620, s'ils sont projetés sur les séquences de Cry1, dans lequel les domaines sont dérivés d'au moins deux protéines Cry différentes, le domaine I est le domaine I de Cry1Ia ou Cry1Ba ou un peptide compris par ledit domaine et ayant au moins 80% de la séquence consécutive de celui-ci ou un peptide ayant une séquence d'acides aminés qui est à au moins 85% identique à la séquence dudit domaine, le domaine II est le domaine II de Cry1Ia ou un peptide compris par ledit domaine et ayant au moins 80% de la séquence consécutive de celui-ci ou un peptide ayant une séquence d'acides aminés qui est à au moins 85% identique à la séquence dudit domaine, et le domaine III est le domaine III de Cry1Ba ou un peptide compris par ledit domaine et ayant au moins 80% de la séquence consécutive de celui-ci ou un peptide ayant une séquence d'acides aminés qui est à au moins 85% identique à la séquence dudit domaine.

2. Fragment de toxine selon la revendication 1 comprenant une séquence d'acides aminés d'environ l'acide aminé 20 à environ l'acide aminé 641 telle que montrée à la SEQ ID NO : 2 ou une séquence d'acides aminés d'environ l'acide aminé 20 à environ l'acide aminé 632 telle que montrée à la SEQ ID NO : 4.

3. Toxine hybride comprenant le fragment selon l'une quelconque des revendications précédentes.

4. Toxine selon la revendication 3 comprenant une séquence d'acides aminés telle que montrée à la SEQ ID NO : 2 ou à la SEQ ID NO : 4.

5. ADN recombinant comprenant une séquence codant pour une protéine ayant une séquence d'acides aminés des protéines selon l'une quelconque des revendications 1 à 4.

6. ADN recombinant selon la revendication 5 comprenant la séquence telle que montrée à la SEQ ID NO : 1 ou 3 ou ADN similaire à celui-ci codant pour une protéine sensiblement similaire.

7. ADN recombinant selon la revendication 5 comprenant la séquence de nucléotides d'environ le nucléotide 170 à environ 1929 dans la SEQ ID NO : 1 ou d'environ 147 à environ 1896 dans la SEQ ID NO : 3.

8. ADN recombinant selon l'une quelconque des revendications 5 à 7 qui est modifié en ce que les motifs d'instabilité de l'ARNm ou les signaux de polyadénylation connus sont enlevés et/ou les codons qui sont préférés par l'organisme dans lequel l'ADN recombinant doit être inséré sont utilisés de sorte que l'expression de l'ADN ainsi modifié dans ledit organisme donne une protéine sensiblement similaire à celle obtenue par l'expression de l'ADN recombinant non modifié dans l'organisme dans lequel les composants protéiques de la toxine ou des fragments de toxine hybride sont endogènes.

9. Séquence d'ADN qui est complémentaire de celle qui s'hybride sous des conditions stringentes avec l'ADN selon l'une quelconque des revendications 5 à 8.

10. Vecteur contenant une séquence d'ADN selon l'une quelconque des revendications 5 à 9.

11. Végétal ou micro-organisme qui inclut, et permet l'expression de, l'ADN selon l'une quelconque des revendications 5 à 9 ou du vecteur selon la revendication 10.

12. Végétaux transformés avec l'ADN recombinant selon l'une quelconque des revendications 5 à 9, descendance de tels végétaux qui contient l'ADN stablement incorporé et pouvant être hérité de manière mendélienne, et/ou graines de tels végétaux et de telle descendance.

13. Protéine dérivée de l'expression de l'ADN selon l'une quelconque des revendications 5 à 9 et protéine insecticide produite par l'expression de l'ADN recombinant dans les végétaux selon la revendication 12.

14. Composition insecticide comprenant une ou plusieurs des protéines selon l'une quelconque des revendications 1 à 4 et 13.

15. Procédé pour lutter contre les insectes qui comprend de les exposer aux protéines ou compositions selon l'une quelconque des revendications 1 à 4, 13 et 14 ou au micro-organisme selon la revendication 11.
